# EUROPEAN PATENT SPECIFICATION

(11) **EP 3 638 807 B1**
(45) Date of publication and mention of the grant of the patent: **30.03.2022**
(21) Application number: 18728664.6
(22) Date of filing: 11.06.2018
(51) Int. Cl.: C12Q 1/68, A61K 39/00, C12Q 1/6886

(54) **MIRNA-574-5P AS A BIOMARKER FOR STRATIFICATION OF PROSTAGLANDIN E-DEPENDENT TUMORS**
MIRNA-574-5P ALS BIOMARKER ZUR STRATIFIZIERUNG VON PROSTAGLANDIN-E-ABHÄNGIGEN TUMOREN
MIRNA-574-5P UTILISÉ COMME BIOMARQUEUR POUR LA STRATIFICATION DE TUMEURS DÉPENDANTES DE LA PROSTAGLANDINE E

(30) Priority: 12.06.2017 EP 17175535
(43) Date of publication of application: 22.04.2020
(73) Proprietor: Fraunhofer-Gesellschaft zur Förderung der angewandten Forschung e.V., 80686 München (DE)
(72) Inventor: STEINHILBER, Dieter, 61389 Schmitten (DE); SAUL, Meike Julia, 64293 Darmstadt (DE); SUESS, Beatrix, 64287 Darmstadt (DE); BAUMANN, Isabell, 63500 Seligenstadt (DE); JAKOBSSON, Per-Johan, 16860 Bromma (SE)
(74) Representative: Altmann Stößel Dick Patentanwälte PartG mbB
(86) International application number: PCT/EP2018/065318
(87) International publication number: WO 2018/228978

(56) References cited:
- WO-A2-2011/135459
- WO-A2-2013/082624
- US-A1- 2012 016 002
- RUI ZHOU ET AL: "Tumor invasion and metastasis regulated by microRNA-184 and microRNA-574-5p in small-cell lung cancer", ONCOTARGET, vol. 6, no. 42, 29 December 2015 (2015-12-29), pages 44609-44622, XP055426609, DOI: 10.18632/oncotarget.6338
- FORSBERG L ET AL: "Human glutathione dependent prostaglandin E synthase: gene structure and regulation", FEBS LETT, ELSEVIER, AMSTERDAM, NL, vol. 471, no. 1, 7 April 2000 (2000-04-07) , pages 78-82, XP004336989, ISSN: 0014-5793, DOI: 10.1016/S0014-5793(00)01367-3

## Description

The present disclosure relates to the field of diagnostic methods. More specifically, it relates to a method for identifying a subject suffering from a prostaglandin E-dependent tumor as being susceptible to a therapy with an inhibitor of prostaglandin E formation comprising the steps of determining in a test sample of the subject the amount of miRNA-574-5p, comparing the determined amount to a reference, and identifying a subject suffering from a prostaglandin E-dependent tumor as being susceptible to a therapy with an inhibitor of prostaglandin E formation based on said comparison. Moreover, the disclosure also encompasses the use of miRNA-574-5p or a detection agent that specifically binds thereto in a sample of a subject suffering from a prostaglandin E-dependent tumor for identifying the subject as being susceptible to a therapy with an inhibitor of prostaglandin E formation and a device as well as a kit for carrying out the method of the disclosure.

Non-small cell lung cancer (NSCLC) is the most common form of lung cancer and accounts for approximately 80% of all cases. Despite recent advances in the management and diagnosis of NSCLC, the 5-year survival rate is about 18%. However, variability in the response to targeted therapies has been reported and may involve the activation of compensatory signaling pathways of cell proliferation and survival.

One of these alternative mechanisms involves the transactivation of epidermal growth factor receptor by prostaglandin (PG) E₂ (Pai, 2002). This prostanoid plays a central role in the development and progression of various types of cancer, including lung cancer tumor. In inflammation and cancer, the major pathway involved in enhanced biosynthesis of PGE₂ is the coordinated activity of cyclooxygenase (COX)-2 which converts arachidonic acid to prostaglandin H₂ (PGH2), and microsomal prostaglandin E synthase-1 (mPGES-1) which catalyzes PGH2 isomerization to PGE₂ (Jakobsson, 1999). Both COX-2 and mPGES-1 are commonly upregulated in NSCLC, but their relative degree of overexpression varies, thus, indicating different underlying regulatory mechanisms.

PGE2 biosynthesis can be inhibited by nonsteroidal anti-inflammatory drugs (NSAIDs) which comprise selective and nonselective COX inhibitors (Patrignani, 2015). A significant increase in survival was found, however, only in individuals with tumors that overexpressed COX-2 or were characterized by reduced PGE₂ biosynthesis in vivo (Hughes, 2008).

An alternative approach to prevent enhanced PGE₂ biosynthesis might involve the use of drugs affecting only mPGES-1 activity such as the selective mPGES-1 inhibitor Compound III (CIII, a benzoimidazole) which inhibits both murine and human recombinant mPGES-1 with marginal inhibitory effects on other prostanoid synthases (Leclerc, 2013). This drug also profoundly inhibits PGE₂ biosynthesis in A549 cells, a human pulmonary epithelial cell line derived from a lung adenocarcinoma as well as in LPS-stimulated human whole blood, i.e., in the presence of plasma proteins (Leclerc, 2013).

The therapeutic value of NSAIDs and inhibitors of PEG synthases is discussed in the field of oncology with great controversy since not all patients appear to be suitable for said treatments.

Dysregulated expression of microRNAs (miRNAs), a class of small non-coding RNAs, has occupied a center stage in the development and progression of lung cancer. Among them, miR-574-5p has been identified and represents a biomarker for early-stage NSCLC14. Furthermore, this miRNA enhances tumor progression via downregulation of the checkpoint suppressor gene 1 in human lung cancer (Li, 2012). The overexpression of miR-574-5p may distinguish patients with metastatic lung cancer and promotes the migration and invasion of NSCLC cell lines (Zhou, 2016).

Zhou et al. (2015), Oncotarget 6(42):44609 reports tumor invasion and metastasis regulated by microRNA-184 and microRNA-574-5p in small-cell lung cancer. WO 2013/082624 A2 describes methods of preventing cancer, treating cancer, inhibiting tumor growth, predicting patient outcomes, optimizing dosage, and monitoring the efficacy of treatment using certain microRNAs and messenger RNAs as indicators. Forsberg et al. (2000), FEBS Lett 471(1):78 reports the gene structure and regulation of human glutathione dependent prostaglandin E synthase. WO 2011/135459 A2 describes methods, devices, and kits for predicting the sensitivity of a patient to a compound or medical treatment based on microRNAs. US 2012/016002 A1 relates to a patient selection process for identifying individuals for treatment of cancer, inflammation, pain, and/or related conditions with a COX-2 selective inhibitor based on PGE2 metabolite levels.

However, so far no means are available that would allow stratifying tumors such as NSCLC for responding or non-responding to the treatment with therapeutic drugs such as NSAIDs or PEG synthase inhibitors. Nevertheless, such stratification means would be highly desirable.

The present invention, thus, relates to a method for identifying a subject suffering from a prostaglandin E-dependent tumor as being susceptible to a therapy with an inhibitor of prostaglandin E formation comprising the steps of:
a) determining in a test sample of the subject the amount of miRNA-574-5p;
b) comparing the determined amount to a reference; and
c) identifying a subject suffering from a prostaglandin E-dependent tumor as being susceptible to a therapy with an inhibitor of prostaglandin E formation based on said comparison.

The method of the present invention, preferably, is an *ex vivo* method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a) or a computer-implemented comparison and/or diagnosis algorithm running on a data processing device in steps (b) or (c).

The term "subject" as used herein refers to an animal, preferably a mammal, such as a laboratory animal, e.g. a mouse or rat, a farming animal, e.g., a pig, a cow, a horse or a goat or a pet, e.g., a cat or dog. More preferably, the subject is a human. The subject in accordance with the present invention shall suffer from a prostaglandin E-dependent tumor as described elsewhere herein.

The term "prostaglandin E-dependent tumor" as used herein refers to a tumorous cancer entity the growth and/or development thereof being dependent on the formation of prostaglandin E. Preferably said tumor is selected from the group consisting of: lung cancer, colon carcinoma, breast cancer, prostate cancer, head and neck cancers, papillary thyroid carcinoma, low and high risk gliomas, neuroblastoma, medulloblastoma, cervix carcinoma, brain tumors and stomach cancer. More preferably, said tumor is lung cancer and, most preferably, non-small cell lung cancer.

The term "identifying" as used herein refers to assigning a feature to the subject, i.e. the feature of being susceptible to a therapy with an inhibitor of prostaglandin E formation, or not. As will be understood by those skilled in the art, such an assignment is usually not intended to be correct for 100% of the subjects to be investigated. The term, however, requires that the assignment, i.e. the identification, is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "susceptible to a therapy" as used herein means that a subject is capable of responding to a therapy as referred to in accordance with the present invention. A response of the subject, preferably, is a therapeutic response. A therapeutic response means that the subject shows amelioration, cure or other improvement of a disease or condition or symptoms associated with said disease or condition. Moreover, the term may also mean that a subject receiving a therapy dose not adversely react on said therapy. Typically, such an adverse reaction results in harmful side effects associated with the therapy.

The term "inhibitor of prostaglandin E formation" refers to a compound or composition that is capable of preventing or reducing the formation of prostaglandin E *in vivo.* Preferably, the reduction is a statistically significant reduction. The inhibitor of prostaglandin E formation can be derived from various classes of chemical molecules or compositions thereof. It may be an artificially synthesized compound or composition or be naturally derived, e.g., from plants, plant parts or microorganisms. Whether a compound or composition is capable of preventing or reducing the formation of prostaglandin E *in vivo* can be determined by tests known to the skilled person and described in more detail, inter alia, in the accompanying Examples, below. Preferably, the inhibitor of prostaglandin E formation is an inhibitor or antagonist of microsomal prostaglandin E-synthase-1. Moreover, non-steroidal anti-inflammatory drugs have also been reported to interfere with prostaglandin E formation. Thus, preferably, the inhibitor of prostaglandin E formation is a non-steroidal anti-inflammatory drug (NSAID) and/or an inhibitor of microsomal prostaglandin E-synthase-1 (mPGES1 inhibitor). More preferably, said NSAID is selected from the group consisting of: selective COX-2 inhibitors, preferably, parecoxib, etoricoxib, or celecoxib, COX1-selective inhibitors, preferably, low dose Aspirin, and non-selective COX1/2 inhibitors, preferably, naproxen, ibuprofen or diclofenac. More preferably, said mPGES1 inhibitor is selected from the group consisting of: MK-886 derivatives, phenanthrene or benzo imidazoles, biarylimidazoles, pirinixic acid derivatives, 2-mercaptohexanoic acid, licofelone derivatives, benzo(g)indol-3-carboxylate, oxicams, trisubstituted ureas, and carbazole benzamides.

The term "test sample" as used herein refers to a tissue sample or a body fluid sample. Preferably, said tissue sample shall comprise tumor tissue of the prostaglandin E-dependent tumor and said body fluid sample shall be suspected to comprise miRNA-574-5p (also referred to as microRNA-574-5p). More preferably, said body fluid sample is selected from the group consisting of: whole blood, serum, plasma, urine and lymphe.

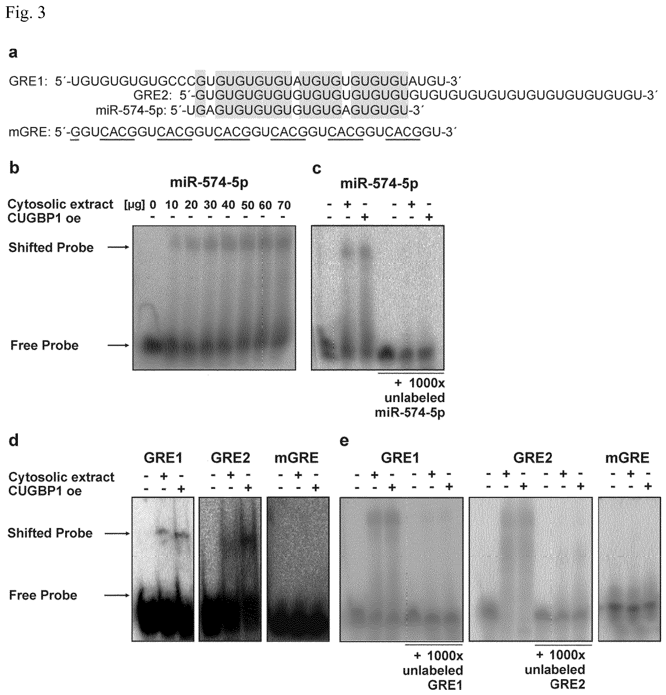

The term "miRNA-574-5p" or "miR-574-5p" refers to a miRNA having a nucleic acid sequence as shown in SEQ ID NO: 3 or shown in Fig. 3, below. MicroRNAs are small non-coding, single stranded RNAs which are capable of RNA gene silencing and post-transcriptional regulation of gene expression. Typically, these effects are elicited by base pairing with a target sequence within an mRNA molecule and subsequent cleavage of the mRNA, shortening of the poly-A tail of the mRNA and destabilization thereby and/or less efficient translation of the mRNA by ribozymes. MicroRNAs are generated within the cell from precursor molecules, the so called pre-miRNAs which are self-complementary, single stranded RNAs having a stem-loop structure. These pre-miRNAs result from further precursors, the pri-miRNAs or from pre-mRNAs.

Determining the amount of miRNA-574-5p relates to measuring the amount or concentration, preferably semi-quantitatively or quantitatively. Measuring can be done directly or indirectly. Direct measuring relates to measuring the amount or concentration of the miRNA-574-5p based on a signal which is obtained from the miRNA-574-5p itself and the intensity of which directly correlates with the number of molecules of the miRNA-574-5p present in the sample. Such a signal - sometimes referred to herein as intensity signal -may be obtained, e.g., by measuring an intensity value of a specific physical or chemical property of the miRNA-574-5p. Indirect measuring includes measuring of a signal obtained from a secondary component (i.e. a component not being the miRNA-574-5p itself) or a biological read out system, e.g., measurable cellular responses, ligands, labels, or enzymatic reaction products.

In accordance with the present invention, determining the amount of miRNA-574-5p can be achieved by all known means for determining the amount of a miRNA in a sample. Said means comprise nucleic acid analyzing devices and methods which may utilize labelled molecules that specifically bind to the miRNA-574-5p in various assay formats. Said assays will develop a signal which is indicative for the presence or absence of the miRNA-574-5p. Moreover, the signal strength can, preferably, be correlated directly or indirectly (e.g. reverse- proportional) to the amount of miRNA-574-5p present in a sample. Typically, hybridization- or PCR-based techniques for nucleic acid analysis are applied for quantitatively determining the amount of a miRNA. Said techniques, preferably, use nucleic acid molecules that specifically hybridize to the target nucleic acid, i.e. the miRNA, as specific detection agents. Moreover, the PCR-based techniques also use nucleic acid molecules as primers for specifically amplifying the target miRNA and, thus, further specific detection agents. Nucleic acid molecules that specifically bind to the target miRNA can be derived from the nucleic acid sequence of miRNA-574-5p by the skilled person without further ado. Such sequences, typically, have a complementary nucleic acid sequence to the target sequence. Moreover, oligonucleotides suitable as primer nucleic acids can also be derived from the miRNA sequence to be amplified by the skilled artisan without further ado.

Further suitable methods comprise measuring a physical or chemical property specific for the miRNA-574-5p such as its precise molecular mass or NMR spectrum. Said methods comprise, preferably, biosensors, optical devices coupled to nucleic acid analyzers, biochips and, in particular, nucleic acid arrays, immunoassays, analytical devices such as mass- spectrometers, NMR- analyzers or chromatography devices.

Preferably, the amount of miRNA-574-5p can be determined by a nucleic acid analysis technology and, more preferably, by a PCR-based technology. In particular, the amount of miRNA-574-5p can be determined by real-time quantitative reverse transcription PCR (qRT-PCR), most preferably, carried out as described in the accompanying Examples, below.

Preferably, digital PCR technologies may be used for the quantification of miRNA-574-5p as well. To this end, the miRNA is also reverse transcribed and subsequently analyzed by digital PCR technologies.

Also preferably, the amount of miRNA-574-5p may be determined by *in situ* hybridization technologies were a labeled probe is used in order to determine the miRNA molecules in a tissue sample. Quantification may be carried out by optically determining a label, such as a fluorescent or chemiluminescent label coupled to the detection probe.

Yet preferably, the amount of miRNA-574-5p may also be determined by hybridization techniques using a target specific probe which subsequently binds to linker and enhancer probes such as the RNAscope technology. This technology may also be used in an in situ approach on tissue samples. In order to evaluate the amount of bound labeled probes, flow-through cytometry may be applied.

Also preferably, determining the amount of miRNA-574-5p may comprise the step of measuring a specific intensity signal obtainable from the miRNA-574-5p in the sample. As described above, such a signal may be the signal intensity observed at an m/z variable specific for the miRNA-574-5p observed in mass spectra or a specific NMR spectrum.

The term "amount" as used herein encompasses the absolute amount of miRNA-574-5p, the relative amount or concentration of miRNA-574-5p as well as any value or parameter which correlates thereto or can be derived therefrom. Such values or parameters comprise intensity signal values from all specific physical or chemical properties obtained from the said miRNA-574-5p molecules by direct measurements. Moreover, encompassed are all values or parameters which are obtained by indirect measurements specified elsewhere in this description, e.g., response levels determined from biochemical or biological read out systems in response to the miRNA-574-5p or intensity signals obtained from specifically bound ligands. It is to be understood that values correlating to the aforementioned amounts or parameters can also be obtained by all standard mathematical operations.

The term "comparing" as used herein encompasses comparing the amount of miRNA-574-5p comprised by the test sample with an amount of a suitable reference specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. The reference is to be chosen so that either a difference or a similarity in the compared amounts allows identifying those test subjects which belong into the group of subjects being susceptible to a therapy with an inhibitor of prostaglandin E formation.

Accordingly, the term "reference" as used herein refers to a reference amount which allows assessing whether a subject suffering from a prostaglandin E-dependent tumor is susceptible to a therapy with an inhibitor of prostaglandin E formation, or not. Accordingly, the reference may, e.g., be derived from a subject or a group of subjects suffering from a prostaglandin E-dependent tumor known to be susceptible to a therapy with an inhibitor of prostaglandin E formation, a non-tumor tissue sample or from a subject or a group of subjects suffering from a prostaglandin E-dependent tumor known not to be susceptible to a therapy with an inhibitor of prostaglandin E formation or, alternatively, a healthy subject or group of healthy subjects. The reference amount may be used to define and establish a threshold amount. The threshold amount, preferably, allows for a rule-in and/or a rule-out diagnosis. The reference amount applicable for an individual subject may vary depending on various physiological parameters such as age, gender, or subpopulation, as well as on the means used for the determination of the miRNA referred to herein. A suitable reference amount may be determined from a reference sample to be analyzed together, i.e. simultaneously or subsequently, with the test sample.

Preferably, said reference is derived from a subject or a group of subjects suffering from a prostaglandin E-dependent tumor known to be susceptible to a therapy with an inhibitor of prostaglandin E formation. More preferably, an essentially identical or increased amount of miRNA-574-5p in the test sample is indicative for a subject being susceptible to a therapy with an inhibitor of prostaglandin E formation and wherein a decreased amount of miRNA-574-5p in the test sample is indicative for a subject being not susceptible to a therapy with an inhibitor of prostaglandin E formation.

Also preferably, said reference is derived from (i) a non-tumor tissue sample or from (ii) a subject or a group of subjects suffering from a prostaglandin E-dependent tumor known not to be susceptible to a therapy with an inhibitor of prostaglandin E formation or (iii) a healthy subject or group of healthy subjects. More preferably, an essentially identical or decreased amount of microRNA-574-5p in the test sample is indicative for a subject being not susceptible to a therapy with an inhibitor of prostaglandin E formation and wherein an increased amount of microRNA-574-5p in the test sample is indicative for a subject being susceptible to a therapy with an inhibitor of prostaglandin E formation.

Advantageously, it was observed in accordance with the studies underlying the present invention that in lung cancer patients, miR-574 expression is strongly upregulated in cancer tissue compared to normal tissue. The studies further aimed to investigate: (i) whether miR-574-5p overexpression in tumor cells influences PGE₂ biosynthesis and unravel the molecular mechanisms involved, (ii) the functional consequences of miR-574-5p overexpression in tumor cells on xenograft tumor growth in immunodeficient mice and (iii) whether the inhibition of miRNA-induced mPGES-1 activity by a PEG synthase inhibitor puts the brake on lung tumor cell growth. Thereby, a potential link between the microRNA and PEG mediated lung cancer formation should be elucidated. MicroRNAs (miRs) are important post-transcriptional regulators of gene expression. Besides their well-characterized inhibitory effects on mRNA stability and translation, miRs can also activate gene expression. In the studies underlying the present invention, a non canonical function of miR-574-5p was identified. It acts as RNA decoy to CUG-binding protein 1 (CUGBP1) and antagonizes its functions. A strong upregulation of miR-574-5p in human lung tumors was observed. This upregulation was associated with an enhanced mPGES-1-dependent biosynthesis of PGE₂, a bioactive lipid mediator that plays a predominant role in lung tumor progression. MiR-574-5p induced mPGES-1 expression by preventing CUGBP1 binding to its 3'UTR which lead to an enhanced PGE₂ formation and tumor growth in vivo. Thanks to this newly discovered association, miR-574-5p was revealed as a useful biomarker to select lung tumor patients who will respond to the pharmacological inhibition of PGE₂. Specifically, it was shown that miR-574-5p is strongly upregulated in lung cancer tissue. When overexpressed in A549 cells, miR-574-5p induced a strong increase in PGE₂ formation. In a xenograft mouse model where injection of A549 cells overexpressing miR-574-5p into nude mice, a highly significant induction of tumor growth and PGE-M levels in urine compared to A549 control cells was observed which was blocked with the selective mPGES-1 inhibitor. The data suggest that miR-574-5p exhibits oncogenic properties and promotes tumor growth via the induction of mPGES-1 derived PGE₂ synthesis. Advantageously, it was found that CUGBP1 inhibits mPGES-1 expression via alternative splicing and translation repression by binding to the GREs in the 3'UTR of the mPGES-1 gene. Furthermore, miR-574-5p can act as decoy by binding to CUGBP1 thereby preventing its regulatory function. Thus, a novel non canonical function of miR-574-5p was revealed, namely to act as a decoy regulating RNA binding protein CUGBP1. Thereby, it could be shown that the balance of CUGBP1 and miR-574-5p regulates cell proliferation, tumor growth and mPGES-1-dependent PGE₂ synthesis. Based on these results in vivo, miR-574-5p apparently plays a major role in the regulation of mPGES-1 dependent PGE2 release in lung cancer and, therefore, offers a novel therapeutic target in treatment of lung cancer and other PEG dependent tumors. Interestingly, upregulation of PGE₂ synthesis by miR-574-5p only involves mPGES-1 whereas COX-2 is not regulated by CUGBP1 and miR-574-5p. The newly discovered link between miR-574-5p overexpression and PGE₂-mediated growth of lung cancer cells in vivo suggests that pharmacological inhibition of PGE₂ formation with NSAIDs or mPGES-1 inhibitors might be of considerable therapeutic value for patients with NSCLC with high miR-574-5p levels.

It is to be understood that the definitions and explanations of the terms made above and below apply accordingly for all embodiments described in this specification and the accompanying claims.

The present invention further relates to the use of microRNA-574-5p or a detection agent that specifically binds thereto in a sample of a subject suffering from a prostaglandin E-dependent tumor for identifying the subject as being susceptible to a therapy with an inhibitor of prostaglandin E formation.

The present invention also relates to a device according to claim 14. The present disclosure also relates to a device adapted for carrying out the method of the invention comprising
a) an analyzing unit comprising a detection agent which specifically binds to microRNA-574-5p adapted for determining the amount of said microRNA-574-5p in a sample of a subject suffering from a prostaglandin E-dependent tumor; and
b) an evaluation unit for comparing the determined amount with a reference whereby the subject can be identified as being susceptible to a therapy with an inhibitor of prostaglandin E formation, said unit comprising a database with references as defined above and a computer-implemented algorithm for carrying out a comparison.

The term "device" as used herein relates to a system comprising the aforementioned units operatively linked to each other as to allow carrying out the method of the invention. Preferred detection agents that can be used for the analyzing unit are nucleic acid molecules used as probes or primers as disclosed elsewhere herein. The analyzing unit, preferably, comprises said detection agents in immobilized form on a solid support which is to be contacted to the sample comprising microRNA-574-5p, the amount of which is to be determined. Moreover, the analyzing unit may also comprise containers with PCR primers specifically amplifying the microRNA-574-5p. Said PCR primers may be provided from the containers to a reaction area were the test sample is contacted with the primers under conditions which allow for PCR amplification and detection of the miRNA. Moreover, the analyzing unit can also comprise a detector which determines the amount of detection agent which is specifically bound to the miRNA or an amplification product thereof. The determined amount can be transmitted to the evaluation unit. Said evaluation unit comprises a data processing element, such as a computer, with an implemented algorithm for carrying out a comparison between the determined amount and a suitable reference. Suitable references are defined above in context with the method of the present invention. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

Moreover, the present disclosure relates to a kit adapted for carrying out the method of the invention comprising a detection agent which specifically binds to microRNA-574-5p as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Further, the kit shall comprise at least one standard for a reference as defined herein above, i.e. a solution with a pre-defined amount for the microRNA-574-5p representing a reference amount. Such a standard may represent, e.g., the amount of microRNA-574-5p useful as reference as described elsewhere herein.

The present invention also contemplates an inhibitor of prostaglandin E formation for use according to claim 15. The present disclosure also relates to an inhibitor of prostaglandin E formation for use in treating a prostaglandin E-dependent tumor in a subject, wherein said subject exhibits increased amounts of miRNA-574-5p (i) in the tumor tissue compared to non-tumor tissue or (ii) in a body fluid of the subject compared to a reference from a healthy subject or a group of healthy subjects or a subject known to not suffer from a prostaglandin E-dependent tumor or a group of such subjects. Preferably, the subject has been identified by the method of the aforementioned present invention.

The present disclosure also relates to a method of treating a prostaglandin E-dependent tumor by an inhibitor of prostaglandin E formation identifying a subject as being susceptible to a therapy with an inhibitor of prostaglandin E formation by the method of the invention referred to above and administering to said identified subject the inhibitor of prostaglandin E formation in a therapeutically acceptable amount.

### FIGURES

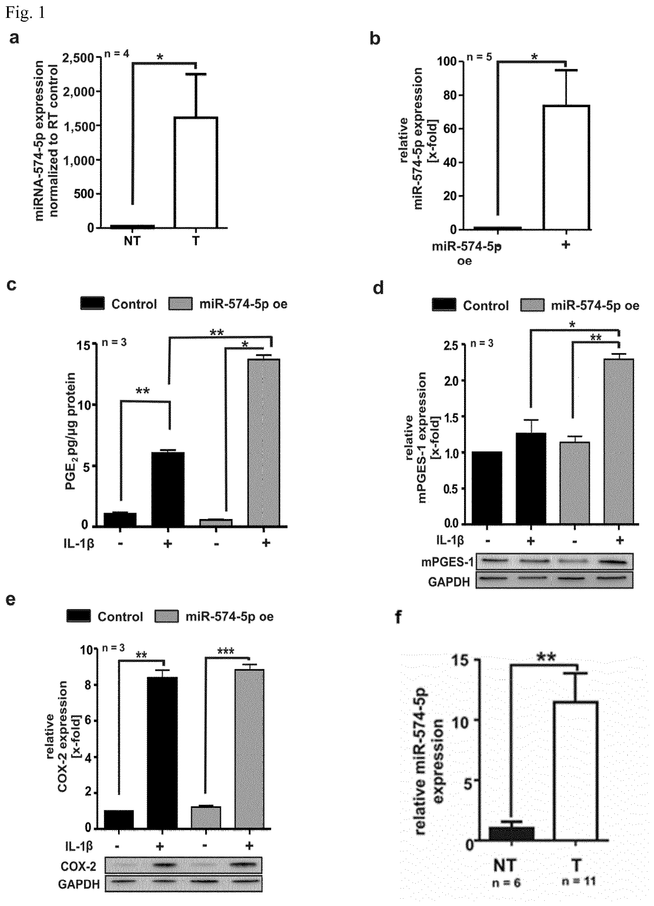

**Figure 1****:** Overexpression of miR-574-5p enhances PGE₂ biosynthesis. (a) Expression of miR-574-5p in non-tumor (NT) and tumor tissue (T) of NSCLC patients (three adenocarcinoma and one squamous carcinoma). The expression was analyzed by qRT-PCR and normalized to miRTC control. Data are shown as mean + SEM. (b) miR 574-5p expression in A549 cells which were stably transduced with miR-574-5p (A549 miR-574-5p oe) or with lentiviral control vector was analyzed by qRT-PCR and normalized to snRNA U6 levels as internal standard. Data are shown as mean + SEM. (c) Overexpression of miR-574-5p increases PGE₂ biosynthesis in IL-1β-stimulated A549 cells. A549 miR574-5p oe or control cells were treated with vehicle (PBS) or IL-1β (5 ng/ml) for 24 h. Then, PGE₂ levels were determined in the conditioned media by a validated radioimmunoassay and normalized to total protein concentration. Data are shown as mean + SEM. (d) mPGES-1 and (e) COX-2 protein expression was determined by Western blot in A549 control and A549 miR-574-5p oe cells incubated with or without IL-1β for 24 h. GAPDH was used as loading control. The relative changes to control t0 (set as 1) are given as the mean + SEM, t-test, ^{∗}p<0.05, ^{∗∗}p<0.01, ^{∗∗∗}p < 0.001. Blots are shown from one representative experiment. (f) Expression of miR-574-5p in non-tumor (NT, n = 6) and tumor tissue (T, n = 11) of NSCLC patients (ten adenocarcinoma and one squamous carcinoma). The expression was analyzed by qRT-PCR and normalized to miRTC control. Data are shown as mean + SEM.

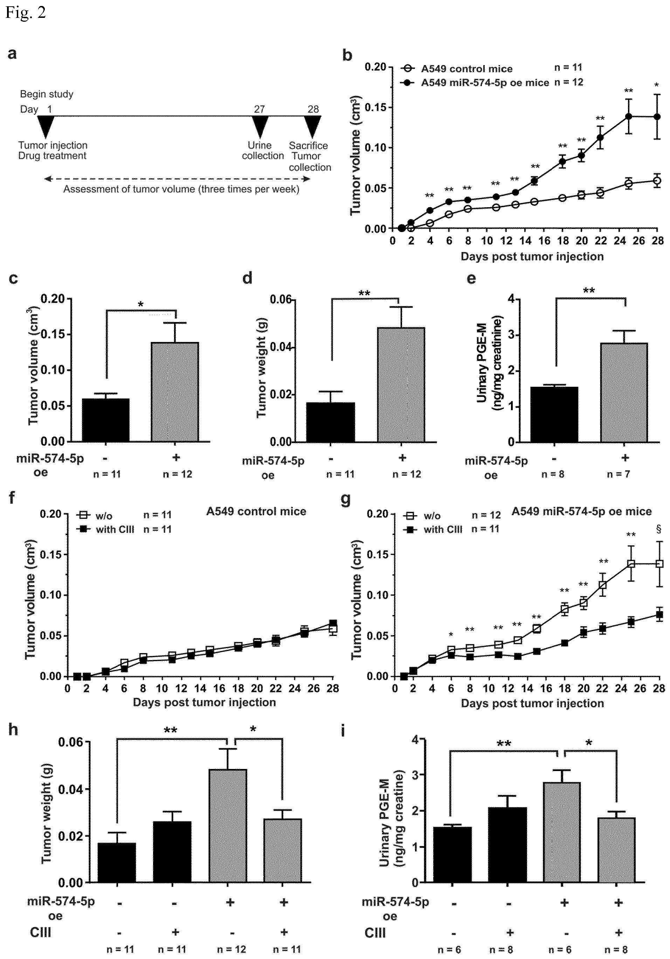

**Figure 2****:** Effects of overexpression of miR-574-5p and mPGES-1 inhibition in A549 cells on tumor growth and systemic PGE2 biosynthesis in A549 xenograft nude mice. (a) Schematic representation of the experimental design. (b) Tumor volume was measured by digital caliper in nude mice inoculated with A549 control or A549 miR-574-5p oe cells and treated with vehicle. Data are shown as mean + SEM. ANOVA analysis and Newman-Keus multiple comparisons test, ^{∗}p<0.05, ^{∗∗}p<0.01. (c) Tumor volume and (d) tumor weight assessed at day 28. Data are reported as mean + SEM, t-test, ^{∗}p<0.05, ^{∗∗}p<0.01. (e) Levels of PGE-M were determined by LC-MS/MS analysis in urine samples collected for 24 h. PGE-M levels are corrected for urinary creatinine levels and data are reported as mean + SEM, t-test, ^{∗∗}p<0.01. Mice injected with (f) A549 control or (g) A549 miR-574-5p oe cells were treated with CIII (50 mg/kg) or vehicle for 4 weeks and tumor volume was assessed. Data are shown as mean ± SEM. ANOVA analysis and Newman-Keus multiple comparisons test. §<0.05, ^{∗}p<0.05, ^{∗∗}p<0.01. (h) Tumor weight assessed at day 28. Data are expressed as mean + SEM, t-test, ^{∗}p<0.05, ^{∗∗}p<0.01. (i) Levels of PGE-M were determined by LC-MS/MS analysis in urine samples collected for 24 h. PGE-M levels are corrected for urinary creatinine levels and data are reported as mean + SEM, t-test, ^{∗}p<0.05, ^{∗∗}p<0.01.
**Figure 3****:** CUGBP1 binds to GU-rich elements of mPGES-1 3'UTR and to mature miR-574-5p. (a) Sequences of GRE1 (SEQ ID NO: 1), GRE2 (SEQ ID NO: 2), miR-574-5p (SEQ ID NO: 3) and mutated mGRE (SEQ ID NO: 4). Bona fide CUGBP1 binding sites are shaded as grey boxes. RefSeq Accession NM_004878.4: GRE1 at position 406-445 and GRE2 at position 1403-1449. (b) Electrophoretic mobility shift assay (EMSA) of radiolabeled miR-574-5p probe incubated with indicated amounts of cytosolic A549 lysates. (c) EMSA of radiolabeled miR-574-5p probe with cytosolic extracts (50 µg protein) of A549 cells without or with CUGBP1 overexpression. Unlabeled miR-574-5p RNA (1000-fold excess) was added in samples as indicated. (d) EMSAs of radiolabeled GRE1, GRE2 and mGRE with cytosolic extracts of A549 cells without or with CUGBP1 overexpression (10 µg protein). (e) EMSAs of radiolabeled GRE1, GRE2 and mGRE with cytosolic A549 cell extracts without or with CUGBP1 oe (50 µg protein). Unlabeled miR-574-5p RNA (1000-fold excess) was added in samples as indicated. RNA-protein complexes were separated by electrophoresis under non-denaturing conditions (6% PAA-Gel). Gels were analyzed on a phosphor imager. In each case, one representative EMSA from three independent experiments is shown.

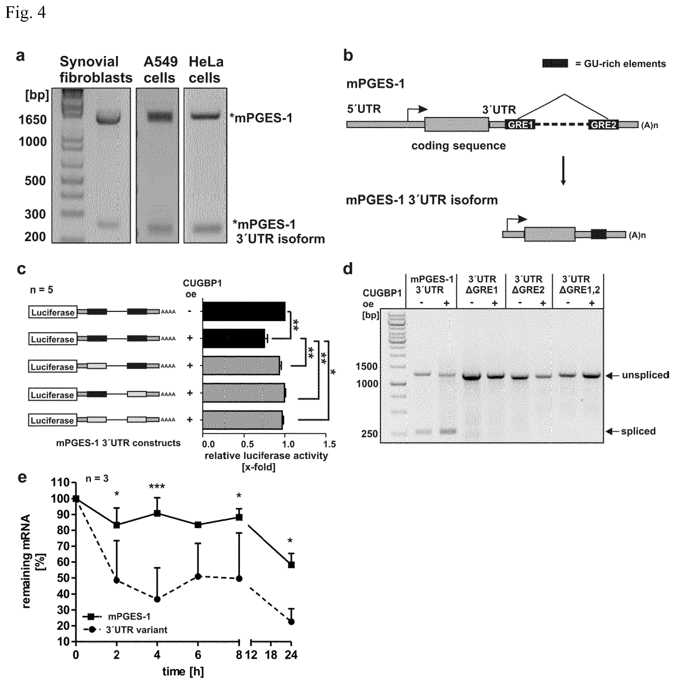

**Figure 4****:** Identification and characterization of a novel mPGES-1 3'UTR mRNA isoform. (a) RT-PCR products obtained with a primer pair covering the mPGES-1 3'UTR in synovial fibroblasts of rheumatoid arthritis patients, A549 and HeLa cells. PCR products were separated on a 1% agarose gel. A representative gel from three independent experiments is shown. (b) Schematic representation of the identified mPGES-1 3'UTR mRNA isoform. GU-rich elements are indicated by black boxes. (c) HeLa cells were transiently transfected with mPGES-1 3'UTR constructs with pCUGBP1 or pCMV-MS control plasmid for 48 h. The black boxes describe GREs within mPGES-1 3'UTR, whereas the grey boxes indicate deleted GREs. After 48 h the luciferase activity was determined and normalized for transfection efficiency using the Dual-GloTM luciferase assay system. The relative changes are given as mean + SEM; t-test, p^{∗} < 0.05; ^{∗∗}p < 0.01. (d) RT-PCR analysis using a primer pair covering mPGES-1 3'UTR in HeLa cells transfected with mPGES-1 3'UTR constructs with and without CUGBP1 oe. RT-PCR products were separated on a 1% agarose gel. A representative gel from three independent experiments is shown. (e) Determination of mPGES-1 and mPGES-1 3'UTR mRNA isoform stability using qRT-PCR. A549 cells were treated with actinomycin D (2 µg/ml) and mPGES-1 as well as mPGES-1 3'UTR isoform mRNA levels were analyzed at the indicated time points. β-Actin was used as control. The relative changes to time point 0 (set as 1) are given as the mean + SEM, 2-way ANOVA, Bonferroni post test, ^{∗}p<0.05, ^{∗∗∗}p<0.001.

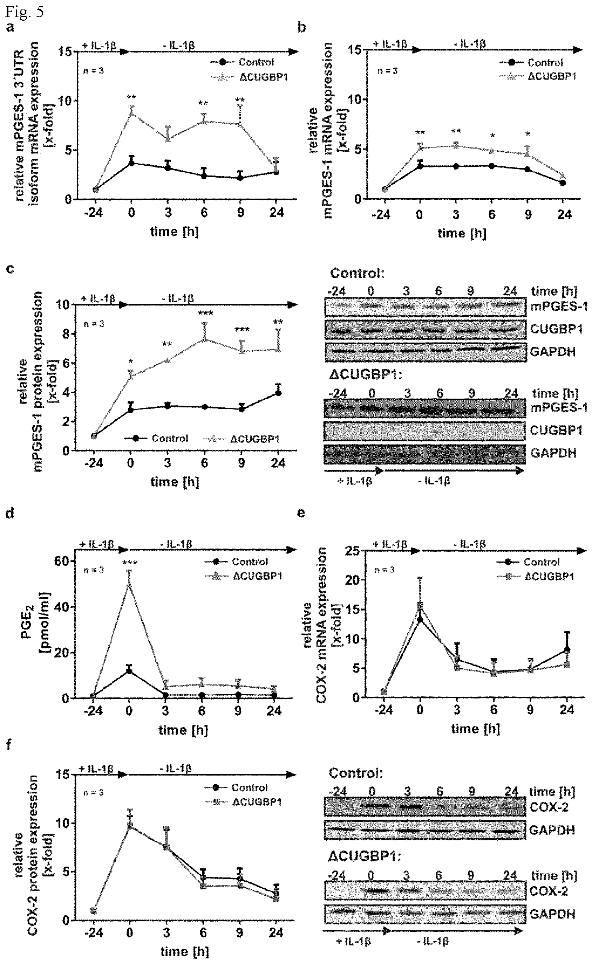

**Figure 5****:** Effect of CUGBP1 knockdown on mPGES-1 and COX-2 expression. A549 cells were transfected with 20 pmol/µl CUGBP1 siRNA or control siRNA and incubated with 5 ng/ml IL-1β for 24 h. Then, cell culture medium was replaced by medium without IL-1β. The cells were cultured for additional 24 h. qRT-PCR analysis of (a) mPGES-1 and (b) mPGES-1 3'UTR isoform mRNA expression was performed at the indicated time points after medium change. β-Actin was used as control. (c) Western blot analysis of mPGES-1 expression. GAPDH was used as loading control. A representative blot from three independent experiments is shown (right panel). (d) LC-MS/MS analysis of PGE2 levels, (e) qRT-PCR analysis of COX-2 mRNA expression. β-Actin was used as control. (f) Western blot analysis of COX-2 protein expression. GAPDH was used as loading control. A representative blot from three independent experiments is shown (right panel). The relative changes to time point -24 h (set as 1) are given as the mean + SEM, 2-way ANOVA, Bonferroni post test, ^{∗}p < 0.05, ^{∗}p < 0.01, ^{∗∗∗}p<0.001.

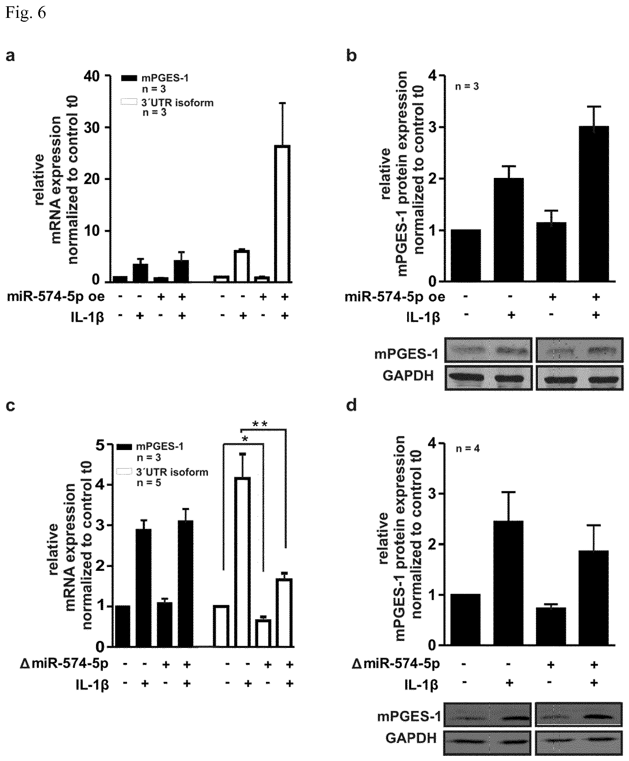

**Figure 6****:** Effect of miR-574-5p overexpression and knockdown on mPGES-1 expression. (a,b) A549 cells without or with miR-574-5p oe were cultured without or with 5 ng/ml IL-1β for 24 h as indicated. (a) qRT-PCR analysis of mPGES-1 and mPGES-1 3'UTR isoform mRNA expression. β-Actin was used as control. (b) Western blot analysis of mPGES-1 protein expression. GAPDH was used as loading control. A representative blot from three independent experiments is shown. (c, d) A549 cells without or with miR-574-5p knockdown were cultured without or with 5 ng/ml IL-1β for 24 h as indicated. (c) qRT-PCR analysis of mPGES-1 and mPGES-1 3'UTR isoform mRNA expression. β-Actin was used as control. The relative changes to control t0 are given as mean + SEM, t-test, ^{∗}p < 0.05. ^{∗∗}p< 0,01. (d) Western blot analysis of mPGES-1 protein expression. GAPDH was used as loading control. A representative blot from four independent experiments is shown.

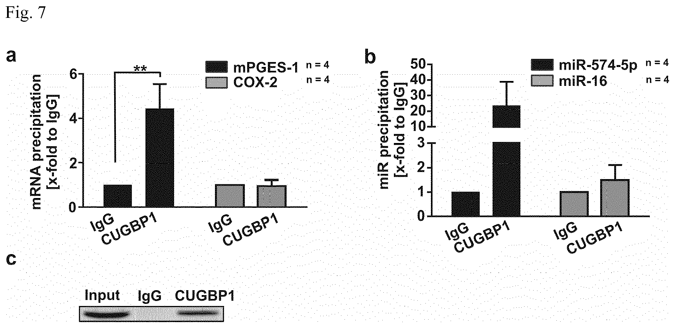

**Figure 7****:** CUGBP1 binds to GU-rich elements of mPGES-1 3'UTR and to mature miR-574-5p. RNA immunoprecipitation (RIP) assay to determine enrichment of mPGES-1 mRNA (a) and miR-574-5p (b) in CUGBP1-IP compared to IgG-IP. COX-2 and miR-16 were used as negative controls. A549 cells were treated with 5 ng/ml IL1-β for 24 h and immunoprecipitation was performed with antibodies against CUGBP1 or normal mouse IgG, respectively. Co-precipitated RNA bound to CUGBP1 was then quantified via qRT-PCR (n = 4). The relative enrichment normalized to IgG is given as mean + SEM, t-test ^{∗∗}p<0.01. (c) Validation of the CUGBP1 immunoprecipitation by Western Blot. 16.6% ± 7.7 (SEM) of the total CUGBP1 were recovered in the immune precipitates. A representative blot of four independent experiments is shown.

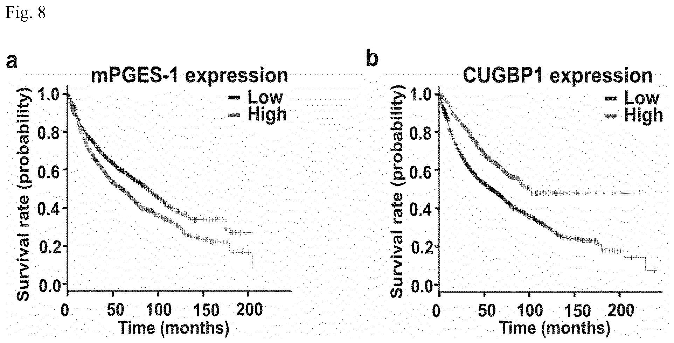

**Figure 8****:** Influence of mPGES-1 and CUGBP1 expression on survival rate of NSCLC patients. (a) High mPGES-1 and (b) low CUGBP1 mRNA expression correlates with a shorter survival rate of NSCLC patients. Kaplan Meier graphs show the overall survival of 1926 lung cancer patients based on "Kaplan Meier plotter" meta-analysis tool (http://kmplot.com/analysis/; version 2015). The overall survival analysis was performed on gene array data from 1926 lung cancer patients. The samples were divided into two groups. In red: patients with expression above the median and in black: patients with expression below the median.

### EXAMPLES

The following Examples shall merely illustrate the invention. They should, whatsoever, not be construed as limiting the scope.

### Example 1: miR-574-5p is upregulated in tumors of NSCLC patients stage II-III

miR-574-5p expression in tumor and non-tumor lung tissue of four NSCLC patients was analyzed. Three tumors of them were histologically categorized as adenocarcinoma and one as squamous carcinoma. A strong increase in miR-574-5p expression in tumors compared to non tumor tissue was found as assessed by qRT-PCR (Fig. 1a). The data were further confirmed by increasing the number of of NSCLC patient samples to measure the miR-574-5p expression in non-tumor tissue (NT, n = 6) and tumor tissue (T, n = 11; ten adenocarcinoma and one squamous carcinoma) of NSCLC patients (Fig. If). These data support the previously suggested important role of miR-574-5p in tumor progression.

### Example 2: Overexpression of miR-574-5p in A549 cells enhances PGE₂ biosynthesis

In order to investigate whether miR-574-5p affects PGE₂ synthesis, A549 cells were used which stably overexpress miR-574-5p (~ 33 fold upregulation) (Fig. 1b). mPGES-1 expression was induced by IL-1β. MiR-574-5p oe leads to a significant increase in PGE₂ biosynthesis compared to A549 control only after induction with IL-1β (Fig. 1c). This effect was associated with increased levels of mPGES-1, but not COX-2 (Fig. 1d,e). The key role of mPGES-1 in the enhanced PGE₂ biosynthesis by overexpression of miR-574-5p is supported by the finding that the biosynthesis of PGF_{2α} (which depends on COX-2 but not mPGES-1) was not affected (data not shown).

Altogether, these results show that miR-574-5p oe promotes mPGES-1-dependent PGE₂ biosynthesis. Next, it was investigated in a xenograft mouse model, whether mPGES-1 induction by miR-574-5p in A549 cells leads to faster growing lung tumors.

### Example 3: The miR-574-5p overexpression in A549 cells increases tumor growth in vivo associated with enhanced systemic biosynthesis of PGE₂

A549 miR-574-5p oe or A549 control cells were injected into the hind flanks of nude mice and tumor formation was monitored up to 4 weeks after injection (Fig. 2a). Tumor volume was evaluated three times a week by using a digital caliper. The mouse group injected with A549 control cells developed 11 tumors, while the mouse group injected with miR-574-5p oe developed 12 tumors which grew in a time-dependent fashion (Fig. 2b). At day 28, the tumor volume averaged 0.059+0.08 cm3 in A549 control (n=11) and 0.138+0.096 in miR-574-5p oe treated animals, respectively (n=12) (Fig. 2c). At sacrifice, tumor weight was significantly higher in A549 miR-574-5p oe-derived tumors (Fig. 2d).

At day 27, the systemic biosynthesis of PGE₂ was evaluated by assessing the levels of PGE-M in 24 h urine. As shown in Figure 2e, average urinary PGE-M levels were significantly higher in mice injected with A549 miR-574-5p oe versus those injected with A549 control cells.

Overall, these results show that the overexpression of miR-574-5p promotes a faster growth of lung cancer cells associated with enhanced biosynthesis of PGE₂ in vivo. These results prompted to verify whether mPGES-1-dependent PGE₂ biosynthesis is responsible for the enhanced tumor growth caused by miR-574-5p oe in xenograft nude mice.

### Example 4: mPGES-1 inhibition reduces the tumorigenic potential of A549 cells in vivo

The selective mPGES-1 inhibitor CIII was used to evaluate whether the stimulation of tumor growth by miR-574-5p in vivo is dependent on increased PGE₂ formation. In order to test this hypothesis, mice which were injected with A549 control (n=11) and A549 miR-574-5p oe cells (n=11) were treated with CIII and tumor progression was evaluated. The results were compared with those obtained in mice injected with A549 control (n=11) or A549 miR-574-5p oe cells (n=12) treated with vehicle only (Fig. 2b). CIII administration did not affect the basal tumor growth of control mice (Fig. 2f), while the compound significantly reduced the time-dependent increase in tumor growth of mice injected A549 miR-574-5p oe cells and tumor progression was comparable with A549 control mice (Fig. 2f,g). At day 28, tumor weight was significantly reduced by CIII administration only in miR-574-5p oe mice (Fig. 2h). The measurement of PGE-M urinary excretion revealed that the drug affected PGE-M levels only in miR-574-5p oe mice by restraining the enhanced PGE₂ biosynthesis which occurred after tumor cell implantation (Fig. 2i). The impact of CIII administration on the systemic biosynthesis of other prostanoids was evaluated. The urinary levels of PGI-M and TX-M were not significantly affected by the drug in miR-574-5p oe as well as in control mice (data not shown). PGD-M levels tended to be higher in miR-574-5p oe mice versus A549 control mice and the CIII administration did not lead to a significant change in PGD-M urinary levels in both mice groups (data not shown).

The data suggest that CIII reduces tumor development only in mice injected with A549 miR-574-5p oe cells by the selective inhibition of PGE₂ biosynthesis without causing a substantial shunting of PGH₂ toward other prostanoids. An immunohistochemical analysis supports that inhibition of mPGES-1 by CIII reduces cell proliferation and angiogenesis (data not shown)

### Example 5: miR-574-5p regulates mPGES-1 expression in a non canonical fashion

The in vivo data indicate that tumor growth is accelerated via miR-574-5p-induced PGE₂ biosynthesis. The mPGES-1 mRNA, however, contains no direct binding site for miR-574-5p and thus is not a target for canonical miRNA silencing. In contrast, overexpression of miR-574-5p increases PGE₂ biosynthesis suggesting an alternative regulatory mechanism than miRNA mediated mRNA silencing.

Recently, a novel miRNA mechanism has been discovered which leads to activation of gene expression. In this case, miR-328 acts as decoy for the RNA binding protein hnRNP E2 counteracting its function as translational repressor. In order to find a similar mechanism for miR-574-5p, GU-rich sequences were found both within the 3' untranslated region (3' UTR) of mPGES1 and in the mature form of miR-574-5p. Such GU-rich sequences (GREs) represent binding sites for CUGBP1 (Fig. 3a). Thus, it was investigated whether miR-574-5p may induce PGE2 synthesis by antagonizing the CUGBP1 function.

Electrophoretic mobility shift assays were performed to determine whether CUGBP1 binds to mature miR-574-5p. Radiolabeled miR-574-5p was incubated with cytosolic extracts from A549 cells and from A549 cells overexpressing CUGBP1 (CUGBP1 oe) by 3.5-fold (data not shown). CUGBP1/miR-574-5p complex formation correlates well with increasing amounts of cytosolic extract (Fig. 3b). Overexpression of CUGBP1 mediates a stronger RNA shift, whereas addition of non-labelled RNA prevents complex formation (Fig. 3c). The shift assays were also performed with two GU-rich elements located in the 3'UTR of mPGES-1 (Fig. 3a). Specific complex formation can only be observed with GRE1 and GRE2 but not with mGRE (Fig. 3d). The signals were eliminated by the addition of excess amounts of non-labeled RNA probes (Fig. 3e). These experiments demonstrate that CUGBP1 specifically binds both the mature miRNA and the 3 'UTR of mPGES-1.

### Example 6: 3'UTR splicing of mPGES-1 is regulated by CUGBP1

The direct binding of CUGBP1 to the GU-rich elements indicates that CUGBP1 is involved in the control of mPGES-1 expression. Bioinformatic analysis of the mPGES-1 3'UTR sequence suggested a putative intron with non canonical GT-TG splice sites which are located within the two GRE elements. Reverse transcription polymerase chain reaction (RT-PCR) analysis in A549 cells, HeLa cells and synovial fibroblasts from rheumatoid arthritis patients verified the existence of the spliced 3'UTR variant in all three cell types (mPGES-1 3'UTR isoform, Fig. 4a). Sequence analysis revealed that the mPGES-1 3'UTR isoform was generated by splicing out the middle part of the 3'UTR that was flanked by the two GREs (Fig. 4b).

To unravel if CUGBP1 influences 3'UTR splicing via binding to the two GRE elements, the mPGES-1 3'UTR was cloned to the firefly luciferase gene open reading frame (Fig. 4c). Luciferase activity was measured in HeLa cells with and without CUGBP1 oe (8,5 fold upregulation). RT-PCR analysis showed that deletion of GRE1, GRE2 or both completely prevents 3'UTR splicing (Fig. 4d) whereas overexpression of CUGBP1 increases the splice process (Fig. 4d). In order to analyze the function of the two GREs, a reporter gene assay was performed which revealed a slight but significant reduction of luciferase activity in response to CUGBP1 oe when both GREs are present but not when one or both GREs were mutated (Fig. 4c).

Further, the stability of both mRNA forms was determined. A549 cells were treated with actinomycin D, a transcriptional inhibitor. RNA was extracted at different times and the respective isoforms were analyzed by qRT-PCR at the indicated time points. As seen in Figure 4e the mRNA stability of mPGES-1 3'UTR isoform is significantly lower than the canonical form.

### Example 7: CUGBP1 regulates PGE₂ synthesis via mPGES-1 expression

To investigate the effect of CUGBP1 on mPGES-1 expression and PGE₂ formation, CUGBP1 expression was knocked down by siRNA (ΔCUGBP1) in A549 cells (data not shown). To assess the CUGBP1-dependent regulation of mPGES-1, A549 cells were treated with IL-1β for 24 h to induce mPGES-1 expression, followed by additional 24 h cultivation in stimulus-free medium. It revealed that IL-1β stimulation leads to an increase in both the 3'UTR isoform and the canoncial form (Fig. 5a,b). ΔCUGBP1 further increases mPGES-1 3'UTR isoform expression (Fig. 5a) and also significantly upregulates mPGES-1 mRNA expression (Fig. 5b), albeit to a lower extent as the mPGES-1 isoform. mPGES-1 protein expression was strongly upregulated in response to ΔCUGBP1, even after change to stimulus-free medium (Fig. 5c). The data demonstrate that CUGBP1 inhibits mPGES1 expression under inflammatory conditions.

Next, it was investigated whether these changes in mPGES-1 expression lead to altered PGE₂ biosynthesis. In A549 cells expressing endogenous CUGBP1, exposed for 24 h to IL-1β, PGE₂ levels were significantly enhanced by simulation with IL-1β for 24 h and returned to baseline levels when the stimulus was removed (Fig. 5d). Of note, the formation of PGE₂ depends on the activity of COX-2 as well as mPGES-1. The reduced levels of PGE₂ detected in A549 cell medium after removal of the inflammatory stimulus are obviously due to COX-2 downregulation both on mRNA and protein level (Fig. 5e,f) whereas mPGES1 levels remained constant (Fig. 5c).

In A549 ΔCUGBP1 cells, PGE₂ formation was significantly upregulated by -50 fold in the presence of IL-1β (Fig. 5d). The COX-2 level was uninfluenced by the ΔCUGBP1 (Fig. 5 e,f). The results show that CUGBP1 influences PGE₂ production in A549 cells by regulating mPGES-1 expression (Fig. 5c), but not COX-2.

### Example 8: CUGBP1 acts as repressor of mPGES-1 translation

Besides its action in the control of splicing, CUGBP1 has also been shown to act as translational repressor. In order to investigate whether CUGBP1 regulates mPGES-1 expression by affecting protein translation, overexpression experiments were performed with CUGBP1 in A549 cells. A significant downregulation of mPGES-1 protein levels was found in response to CUGBP1 oe, but no alteration on the mRNA level (data not shown). These data clearly indicate that CUGBP1 represses mPGES-1 translation.

Recently, it was shown that translational repression of occludin mRNA was due to colocalization of CUGBP1 and tagged mRNA in processing bodies (P-bodies). Therefore, it was hypothesized that CUGBP1 might repress mPGES-1 translation by mPGES-1 mRNA recruitment to P-bodies.

First, the distribution by immunofluorescence staining was investigated and extensive colocalization of CUGBP1 and DCPla (decapping mRNA 1A) was found, a key component of P-bodies. Further, it has been shown that knockdown of the P-body component GW182 (Trinucleotide Repeat Containing 6A) can disrupt P-body formation. Therefore, GW182 (ΔGW182) were depleted to determine whether P-body formation is linked to translational repression of mPGES-1 protein. The mature mPGES-1 mRNA was not affected by ΔGW182, whereas mPGES-1 protein expression was significantly upregulated in A549 cells. These data indicate a role of CUGBP1 as translational repressor of mPGES-1 in combination with P-body formation.

### Example 9: The balance between miR-574-5p and CUGBP1 regulates mPGES-1 expression

It was analyzed how CUGBP1 activity is regulated. Since the expression level, localization and phosophorylation status of CUGBP1 was not affected by IL-1β stimulation, it was hypothesized that an upregulation of miR-574-5p antagonize CUGBP1 function on mPGES1 biosynthesis.

Since we found that CUGBP1 binds to miR-574-5p (Fig. 4B,C), we hypothesized that the strong upregulation of miR-574-5p levels in cancer (Fig. 1A) and after IL-1β treatment of A549 cells (data not shown) maybe is responsible for the upregulation of mPGES-1 expression by counteracting the repressive action of CUGBP1 on mPGES1 biosynthesis.

Therefore, miR-574-5p was overexpressed in A549 cells using miRNA mimics (miR-574-5p oe). miR-574-5p oe resulted an increased expression of the mPGES-1 3'UTR isoform, whereas the canoncial mPGES-1 mRNA was not significantly affected (Fig. 6a). An upregulation of mPGES-1 protein level was observed in response to miR-574-5p oe (Fig. 6b). These results were comparable to those obtained by ΔCUGBP1 (Fig. 5c).

Next, miR-574-5p (ΔmiR-574-5p) expression levels were depleted using LNAs^{™} specific for miR-574-5p. mPGES-1 mRNA expression was not affected by ΔmiR-574-5p, whereas the mPGES-1 3'UTR isoform was significantly reduced in response to ΔmiR-574-5p (Fig. 6c). mPGES-1 protein level was reduced by ΔmiR-574-5p, albeit the reduction did not reach statistical significance (p=0,066, t-test).

### Example 10: Changes in CUGBP1 and miR-574-5p cause opposite effects on A549 cell proliferation

A MTT assay was performed to elucidate the role of miR-574-5p and CUGBP1 in cell proliferation. In this assay, IL1β-induced cell proliferation of A549 cells was enhanced by miR-574-5p oe or CUGBP1 depletion whereas it was reduced by miR-574-5p depletion or CUGBP1 oe (data not shown). These results support the hypothesis that miR-574-5p overexpression translates into enhanced proliferation by counteracting the action of CUGBP1.

### Example 11: General methods

### Cell lines and cell culture conditions

The human lung adenocarcinoma cell line A549 (ATCC Manassas, VA, USA) and the cervical cancer cell line HeLa (DMSZ) were cultured in Dulbecco's modified Eagle medium (DMEM, Life Technologies, ThermoFisher Scientific, Waltham, USA) with 10% (v/v) heat-inactivated fetal bovine serum (FBS, Life Technologies, ThermoFisher Scientific, Waltham, USA), 100 U/ml penicillin 100 µg/ml streptomycin and 1 mM sodium pyruvate (PAA the Cell Culture Company, Cölbe, GER). Synovial fibroblasts of rheumatoid arthritis patients were cultured as described previously32. This study was approved by the Institutional Ethical Committee (Solna, Stockholm, Sweden ethical number 2009/1262-31/3) and is in compliance with all ethical standards and patients' consent according to the Declaration of Helsinki. Cell culture was carried out in a humidified atmosphere of 5% CO₂ at 37°C. A549 cells were stimulated with 5 ng/ml of IL-1β (Sigma-Aldrich, Darmstadt, GER) for 24 h.

### Lung tumor tissue

Lung non-tumor and tumor tissue were collected from 4 different NSCLC patients (three adenocarcinoma and one squamous cell carcinoma). These donor lung tissues were non-transplanted lung tissues of transplant donors. The study protocol for tissue donation was approved by the ethics committee ("Ethik Kommission am Fachbereich Humanmedizin der Justus Liebig Universität Giessen") of the University Hospital Giessen (Giessen, Germany) in accordance with national law and with "Good Clinical Practice/International Conference on Harmonisation" guidelines. Written informed consent was obtained from each patient or the patient's next of kin (AZ 31/93). In a further experiment, Lung non-tumor and tumor tissue samples were collected from 11 different NSCLC patients (ten adenocarcinoma and one squamous cell carcinoma). These donor lung tissues were non-transplanted lung tissues of transplant donors. The study protocol for tissue donation was approved by the ethics committee ("Ethik Kommission am Fachbereich Humanmedizin der Justus Liebig Universität Giessen") of the University Hospital Giessen (Giessen, Germany) in accordance with national law and with "Good Clinical Practice/International Conference on Harmonisation" guidelines. Written informed consent was obtained from each patient or the patient's next of kin (AZ 31/93).

### RNA interference

CUGBP1 and GW182 were transiently depleted using siRNA oligonucleotides. 24 h prior to transfection, A549 cells were seeded at a density of 5×10⁵ cells/well in a 6-well plate. 20 pmol/µl siRNA oligonucleotides were transfected using Lipofectamine 2000^{®} (Invitrogen, Karlsruhe, GER) according to manufacturer's instructions. For GW182 knockdown, MISSION^{®} siRNA SASI_Hs01_00244664 (Sigma-Aldrich, Darmstadt, GER) were used, respectively. For CUGBP1 knockdown a previously published siRNA (5'-GCUGUUUAUUGGUAUGAUU-3'; SEQ ID NO :5) was used for our experiments. As control, a siRNA with an unspecific sequence was used (5'-UCUCUCACAACGGGCAUUU-3'; SEQ ID NO: 6). Cells were harvested 24 h and 48 h after transfection. The efficiency of CUGBP1 and GW182 knockdown was determined by qRT-PCR, Western blot and immunofluorescence staining.

### Overexpression of CUGBP1

24 h prior to transfection, A549 cells were seeded at a density of 4×10⁴ per well in a 24-well plate. 500 ng/well pCUGBP1 or pCMV-MS control plasmid (kindly provided by Julia Weigand and Katrin Kemmerer, Technical University Darmstadt) were tranfected using Lipofectamine 2000^{®} according to the manufacturer's instructions. In the same way, HeLa cells were transfected with 300 ng/well pCUGBP1 of pCMV-MS. The overexpression efficiency was analyzed by Western blot.

### Transfection of miR-574-5p mimic or inhibitor

24 h prior to transfection, A549 cells were seeded at a density of 5×10⁵ per well in a 6-well plate. 20 pmol/µl miRIDIAN miR-574-5p mimics (HMI0794, Sigma-Aldrich, Darmstadt, GER) or negative control (HMC0002, Sigma-Aldrich, Darmstadt, GER) were transfected using Lipofectamine 2000^{®} according to the manufacturer's instructions. In the same way, the A549 cells were transfected with 40 pmol/µl miR-574-5p-LNA^{™} inhibitor (4101451-001) or negative control (199006-001, Exiqon, Kopenhagen, DEN) for the knockdown of miR-574-5p. The transfection efficiency was determined by qRT-PCR.

### Stable overexpression of miR-574-5p in A549 lung cancer cell line

The lentiviral particles Mission^{®} lenti miR-574-5p (HLMIR0794, Sigma-Aldrich, Darmstadt, GER) or Mission^{®} lenti control (NCLMIR002, Sigma-Aldrich, Darmstadt, GER) were used to generate an A549 cell line stably overexpressing miR-574-5p (A549 miR-574-5p-5p oe) and the control cell line (A549 control). 24 h prior to transduction, A549 cells were seeded at a density of 1×10⁵ per well in a 6-well plate. Lentiviral particles were quickly thawed and added to A549 cells at an MOI of 0.83 for spinoculation (875 g, 32°C for 60 min). The transduced cells were grown for 1 day before 1.5 µg/ml puromycin (Sigma-Aldrich, Darmstadt, GER) was added for 4 days to select the transduced clones. The transduction efficiency was verified by qRT-PCR.

### RNA extraction and RT-PCR

Total RNA was extracted with TRIzol reagent (Invitrogen, Karlsruhe, GER) and treated with Turbo DNase (Ambion, ThermoFisher Scientific, Waltham, USA) according to manufacturer's instructions. 1 µg of DNase-treated RNA was used for reverse transcription using the High-Capacity RNA-to-cDNA Kit (Applied Biosystems, ThermoFisher Scientific, Waltham, USA) according to manufacturer's instructions. The first-strand cDNA was finally used as a template for the PCR reaction. The primers mPGES-1 3'UTR_F and mPGES-1 3'UTR_R (Table 1) were used for specific amplification of mPGES-1 3'UTR. The RT-PCR was carried out using 2.5 U Taq polymerase (New England Biolabs, Ipswish, USA) according to the manufacturer's instructions with the addition of 50 µM betaine (Sigma-Aldrich, Darmstadt, GER). Cycle numbers were minimized in order to keep the RT-PCR reaction in the exponential phase and to avoid saturation effects. One tenth of each RT-PCR sample was analyzed by gel electrophoresis on an ethidium bromide-stained agarose gel. QIAquick Gel Extraction Kit (Qiagen, Hilden, GER) was used for gel extraction of the RT-PCR products. PCR CloningPLUS Kit (Qiagen, Hilden, GER) was used to clone the isolated RT-PCR fragments. The selected plasmids were then sequenced.

**Table 1: Primers**

| | |
|---|---|
| mPGES-1 3'UTR F | CCAGCAGCTGATGCCTCCTTG SEQ ID NO :7 |
| mPGES-1 3'UTR_R | GCCCAGCTGGCAGACACTTCC SEQ ID NO :8 |
| mPGES-1 3'UTR_F*_N ot*I | AAGCGGCCGCCCAGCAGCTGATGCCTCCT SEQ ID NO :9 |
| mPGES-1 3'UTR_R *Hind*III | AAAGCTTTGCTGGGCCCAGCTGGCA SEQ ID NO : 10 |
| ΔGRE1_F | TCCCCTTGATGGGGAATCCTTTCTCCTAGACCCGTGACC SEQ ID NO : 11 |
| ΔGRE1R | CGGGTCTAGGAGAAAGGATTCCCCATCAAGGGGA SEQ ID NO : 12 |
| ΔGRE2_F | GCGCGTGTGGGTCTCTGGGCACAGTGGGCCTTTTCTTAGCCCCTTGGATT CCTGC SEQ ID NO :13 |
| ΔGRE2_R | GCAGGAATCCAAGGGGCTAAGAAAAGGCCCACTGTGCCCAGAGAC SEQ ID NO :14 |
| GW182_F | AATCTGGTGCAGCAAACTCC SEQ ID NO :15 |
| GW182_R | AGTGTTTTGTGCAGGGGTTC SEQ ID NO : 16 |
| CUGBP1_F | AAAGTCCTCCCAGGGATGCA SEQ ID NO :17 |
| CUGBP1_R | AGCTTCCTGTCTTCCACTGCAT SEQ ID NO :18 |
| 3'UTR-isoform F | GTGCCCGTGTGTGTGTATGTGTGTGTGTGT SEQ ID NO :19 |
| 3'UTR-isoform R | CCCAGCTGGCAGACACTTCCATTTAATGACT SEQ ID NO :20 |
| COX-2_F | CCGGGTACAATCGCACTTAT SEQ ID NO :21 |
| COX-2 R | GGCGCTCAGCCATACAG SEQ ID NO :22 |
| mPGES1_F | TCCCGGGCTAAGAATGCA SEQ ID NO :23 |
| mPGES1_R | ATTGGCTGGGCCAGAATTTC SEQ ID NO :24 |
| Actin_F | CGGGACCTGACTGACTAC SEQ ID NO :25 |
| Actin R | CTTCTCCTTAATGTCACGCACG SEQ ID NO :26 |
| GRE1_F | AAAAAAGAATTCTAATACGACTCACTATAGGTGTGTGTGTGCCCGTGTG SEO ID NO :27 |
| GRE1_R | TTTTTTCCATGGCCGGCACATACACACACACATACACACACACGGGCAC ACACACACCTATAGT SEQ ID NO :28 |
| GRE2_F | AAAAAAGAATTCTAATACGACTCACTATAGGGTGTGTGTGTGTGTGSEQ ID NO :29 |
| GRE2_R | TTTTACCATGGCCGGCCACACACACACACACACACACACACAC ACACACACACACACACCCTATAGTG SEQ ID NO :30 |

### Quantitative reverse transcription PCR (qRT-PCR)

1 µg of DNase-treated RNA was used for reverse transcription using High-Capacity RNA-to-cDNA Kit (Applied Biosystems, ThermoFisher Scientific, Waltham, USA) according to manufacturer's instructions. Real-time reverse transcription PCR (qRT-PCR) was performed in Applied Biosystems StepOne Plus^{™} Real-Time PCR System (Applied Biosystem, ThermoFisher Scientific, Waltham, USA) using Fast SYBR Green PCR Master Mix (Applied Biosystems, ThermoFisher Scientific, Waltham, USA). 1 µl cDNA (1:2 diluted) was used per reaction. β-Actin was used as endogenous control to normalize variations in cDNA quantities in different samples. The sequences for primer pairs are listed in Table 1.

qRT-PCR based miR-574-5p quantification was performed using miScript system (Qiagen, Hilden, GER), 1 µg DNase digested RNA was used for reverse transcription. qRT-PCR of miR-574-5p was performed using the miR-574-5p specific primer (MS00043617, Qiagen, Hilden, GER). snRNA U6 was used as endogenous control primer (MS00033740, Qiagen, Hilden, GER). miR-574-5p expression of NSCLC tissue samples were normalized to miRNA reverse transcription control using a miRTC primer (MS00000001, Qiagen, Hilden, GER) since snRNA U6, hsa-miR-16 and SNORD72 were no valid controls and because they are regulated in lung cancer37. The miR-574-5p values were normalized to the expression of miRTC control. qRT-PCR was performed according to the manufacturer's instructions.

Fold inductions were calculated using 2(-ΔΔCt) values.

### Plasmid constructs

mPGES-1 3'UTR cDNA was amplified from A549 cells with the primers NotI-mPGES-1 3'UTR_F and HindIII-mPGES-1 3'UTR_R (Tab. S1) and cloned into the pDL Dual Luciferase plasmid38 via NotI and HindIII restriction sites, resulting in the mPGES-1 3'UTR plasmid. Deletions of the two GU-rich elements (GRE-elements) were generated by site-directed mutagenesis PCR using the mPGES-1 3'UTR plasmid as template. For the mutation of the first GU-rich element, ΔGRE1_F and ΔGRE1_R were used resulting in the plasmid ΔGRE1_mPGES-1 3'UTR. The mutation of the second GU-rich element was generated using ΔGRE2_F and ΔGRE2_R resulting in ΔGRE2_mPGES-1 3'UTR. All primer sequences are listed in the Table 1. Plasmids were checked by DNA sequencing.

### Luciferase reporter gene assay

24 h prior to transfection, 4×10⁴ HeLa cells per well were seeded in 24-well plates. 100 ng/well of mPGES-1 3'UTR constructs and 300 ng/well pCUGBP1 and pCMV-MS as control were used for transfection with Lipofectamine 2000^{®} (Invitrogen, Karlsruhe, GER) according to the manufacturer's instructions. After 48 h, cells were assayed for luciferase activity using the Dual-Glo^{™} Stop and Glow Luciferase Assay System (Promega, Madison, USA) with a TECAN infinite M200 reader. Renilla luciferase activity was used to normalize the luciferase activity to the transfection efficacy.

### Western blotting

To obtain a whole cell lysate, A549 cells were lysed in T-PER^{™} tissue protein extraction reagent (Life Technologies, ThermoFisher Scientific, Waltham, USA) for 30 min at 4°C. For separation of the nuclear and cytosolic fraction, cells were lysed in cytosolic extraction buffer containing 10 mM HEPES pH 7.9 (Sigma-Aldrich, Darmstadt, GER), 1.5 mM MgCl2 (Sigma-Aldrich, Darmstadt, GER), 10 mM KCl (Sigma-Aldrich, Darmstadt, GER), protease inhibitor EDTA-free (Roche, Basel, CHE) on ice for 5 min. Samples were centrifuged for 3 min (6,500 rpm). The supernatant was the cytosolic fraction. The pellet was further resuspended in nuclear extraction buffer containing 20 mM HEPES pH 7.9, 1.5 mM MgCl2, 0.42 M NaCl (Sigma-Aldrich, Darmstadt, GER), 0.2 mM EDTA (Sigma-Aldrich, Darmstadt, GER), 25% glycerol (Sigma-Aldrich, Darmstadt, GER) and protease inhibitor EDTA-free (Roche, Basel, CHE) for 15 min at -80°C. Samples were frozen and thawed three times at 37°C. Samples were centrifuged for 15 min at 13,300 rpm. The supernatant was the nuclear fraction. The protein content was determined by Bradford assay (BioRad Laboratories, Hercules, USA). Western blot analysis was performed as previously described39. The membranes were incubated with primary antibodies that recognize mPGES-1 (Cayman-Chemical, Ann Arbor, USA and 40), GAPDH (2118, Cell signaling, Cambrige, UK), CUGBP1 (ab9549, Abcam, Cambrige, UK), phosphorylated CUGBP1 (15903, Abnova, Heidelberg, GER), Lamin a/c (4777S, Cell signaling, Cambrige, UK) and COX-2 (sc-1745, Santa Cruz Biotechnology, Santa Cruz, USA). In experiments involving A549 miR-574-5p-5p oe and A549 control cell lines, the cells were lysed and the analysis of mPGES1, COX-2 and GAPDH was performed as described above.

### Electrophoretic mobility shift assay

Cytoplasmic extracts were prepared by lysing cells for 10 min on ice in a buffer containing 0.2% Nonidet P-40 (Sigma-Aldrich, Darmstadt, GER), 40 mM KCl (Sigma-Aldrich, St. Louis/USA), 10 mM HEPES (pH 7.9) (Sigma-Aldrich, Darmstadt, GER), 3 mM MgCl₂ (Sigma-Aldrich, Darmstadt, GER), 1 mM DTT (Sigma-Aldrich, Darmstadt, GER), 5% glycerol (Sigma-Aldrich, Darmstadt, GER) and protease inhibitor (Roche, Basel, CHE). Nuclei were removed by centrifugation at 13,300 rpm for 2 min and cytoplasmic extracts were immediately frozen on dry ice and stored at -80°C. The protein concentration of the extracts was determined by Bradford assay. GRE1 and GRE2 RNA was in vitro transcribed. Specific primers (Table 1) for GRE1 (GRE1_Fand GRE1_R) and GRE2 (GRE2_F and GRE2_R) were annealed and cloned into the high-copy-number plasmid pHDV. In this way, the HDV ribozyme was added to the 3' end of GRE1 and GRE2 giving homogeneous 3' ends after in vitro transcription. RNAs were end labeled with [γ32P] ATP (6,000 Ci/mmol, Hartmann, Braunschweig, GER) using T4 polynucleotide kinase (Invitrogen, Karlsruhe, GER). The miR-574-5p and mGRE1 RNAs were commercially synthesized by Sigma-Aldrich. EMSA assays were conducted by incubating cytoplasmic extracts with the 32P-labeled RNA probe in a buffer containing 0.2% Nonidet P-40, 40 mM KCl, 10 mM HEPES (pH 7.9), 3 mM MgCl₂, 1 mM DTT, and 5% glycerol and 5 mg/ml heparin sulfate (Sigma-Aldrich, Darmstadt, GER). Each reaction contained 10-70 µg of cytoplasmic protein and 10 fmol of radiolabeled RNA probe in a total volume of 15 µl for 20 min. Each sample was mixed with 3 µl 5x native loading buffer (50%, v/v, glycerol, 0.2% bromophenol blue, 0.5x TRIS-Borate EDTA buffer, Sigma-Aldrich, Darmstadt, GER). The samples were separated by electrophoresis under nondenaturing conditions on 6% polyacrylamide gels (PAA). The gels were analyzed on a Typhoon phosphorimager (Amersham Biosciences, Freiburg, GER).

### MTT cell proliferation assay

For the detection of viable cells, an MTT (3-(4,5-dimethylthiazol-2-yl)-2,5-diphenyltetrazolium bromide) tetrazolium (Carl Roth, Karlsruhe, GER) reduction assay was performed. 24 h prior transfection, A549 cells were seeded at a density of 1×10⁴ cells per well in 96-well plates. For the MTT reduction assay, 20 µl of 5 mg/ml MTT (in PBS) was added to the cells in culture and incubated for 4 h. The medium was removed and 100 µl DMSO (Carl Roth, Karlsruhe, GER) was added. The quantity of formazan was measured by recording changes in the absorbance at 570 nm using a plate reading spectrophotometer. As a reference the wavelength of 630 nm was used to correct for nonspecific background.

### Immunofluorescence staining

A549 cells (5×10⁵) were seeded on cover slides (18 × 18 cm, Carl Roth, Karlsruhe, GER) in 6-well plates and fixed using 4% cold paraformaldehyde (PFA, Carl Roth, Karlsruhe, GER) for 15 min, then washed twice with PBS, followed by 5 min permeabilization with 0.5% Triton X-100 (Sigma-Aldrich, Darmstadt, GER). Cells were blocked with 2% BSA in PBS (Sigma-Aldrich, Darmstadt, GER). Primary antibodies DCPla 1:1000 (ab70522, Abcam, Cambrige, UK), GW182 1:100 (ab66009, Abcam, Cambrige, UK) and CUGBP1 1:500 (ab9549, Abcam, Cambrige, UK) were diluted in blocking buffer and incubated with fixed cells overnight at 4°C. Cells were washed twice with PBS and incubated with secondary antibody goat anti-mouse IgG (Alexa Fluor^{®} 594, abl50116), respectively goat anti-rabbit IgG (Alexa Fluor^{®} 488, ab150077) from Abcam for 2 h at room temperature. Cells were stained with 4',6-diamidine-2'-phenylindole dihydrochloride (DAPI, Sigma-Aldrich, Darmstadt, GER). Images were processed with 20x magnitude using Axio Observer microscope (Zeiss, Jena, GER) with LSM 510 Meta (Zeiss, Jena, GER) image-processing software.

### Prostanoid determination in cell culture supernatants

Prostanoids from cell culture supernatants were extracted from cell culture supernatants by solid phase extraction and analyzed by targeted LC-MS/MS utilizing deuterated internal standards as previously described44.

In experiments performed with A549 miR-574-5p oe and A549 control cell lines, conditioned medium was collected from cultured cells, centrifuged at 10,000 g for 2 min to discard cell debris. The PGE2 and PGF2α level of the supernatants was determined by radioimmunoassay and normalized to the total protein concentration.

### Mouse experiments

Experiments were performed in accordance with the European Communities Council (EEC) Directive of 22 September 2010 (2010/63/EU) and National Ethical Committee. The study was approved by Italian Ministry of Health (MOH), authorization n. 190/2017-PR. CD-1^{®} Nude mice were obtained from Charles River (Milan, ITA). The animals were housed in cages up to five mice each and acclimated for one week under conditions of controlled temperature (20 ± 2°C), humidity (55±10%), and lighting (7:00 a.m. to 7:00 p.m.) before starting experiments. Animals were maintained under specific pathogen-free conditions with food and water provided ad libitum and the animals' health status was monitored daily. All efforts were made to minimize animal suffering and to reduce the number of animals used. Age-matched female (6-week-old to 8-week-old) were used for all experiments. A549 control cells or A549 miR-574-5p-5p oe cells were cultured under standard conditions as described above, harvested by trypsin and resuspended in PBS (5×10⁷/ml). 100 µl of cell suspension (containing 5×10⁶ cells) were subcutaneously injected into both dorsal flanks of mice to establish a model of tumor-bearing mice (Fig 2A). The mPGES-1 inhibitor (CIII, 28575, provided by Novasaid, Stockolm, Sweden) was dissolved in the vehicle containing 1% Tween 80 (Sigma-Aldrich, Darmstadt, GER) and 0.5% carboxymethylcellulose (Sigma-Aldrich, Darmstadt, GER) in 0.9% NaCl solution.

Mice received A549 control cells (n=11) or A549 miR-574-5p-5p oe cells (n=12) and were treated with vehicle, every day, once a day by i.p. injection from tumor cell implantation until sacrifice (day 28, i.e. for 4 weeks) (Fig. 2A). CIII-treated mice received A549 control cells (n=11) or A549 miR-574-5p-5p oe cells (n=11) and the CIII compound (50 mg/kg) was administered once daily by i.p. injection from tumor cell implantation until sacrifice. Tumor growth was monitored three times a week from tumor cell implantation until sacrifice by measuring its diameter with a digital caliper. Tumor volume (TV) was calculated by TV(cm3) = length[D(cm)]×width [d(cm)2]×0.44. Body weight was monitored three times a week until sacrifice and tumor weight (gr) of dissected tumors was assessed at the sacrifice. Mice were sacrificed after 4 weeks from tumor cell implantation, 30 min after the administration of the last dose of drug. At day 28, animals were sacrificed and tumors and lungs were collected and embedded in Tissue-Tek O.C.T. (Sakura, San Marcos, USA), cooled in isopentane and frozen in liquid nitrogen for histology. 7 mm-thick cryostat sections from tumour tissue samples were stained with hematoxylin and eosin and adjacent sections were used for immunohistochemical staining with anti-Ki67 (Cell Signaling, Danvers, MA), anti-VEGF (Bioss, SIAL srl, Rome, ITA) or CD40 (Santa Cruz, DBA, Milan, ITA) antibodies. Cryostat sections were firstly fixed in ice-cold acetone and incubated for 10 min in 3% H₂O₂, washed (3x5 min) in TBS and then incubated in a 3% bovine serum albumin (BSA, Sigma Aldrich, Milan, ITA). Sections were exposed at 37 °C for 1 h to a diluted solution of CD40 (1:30), Ki67 (1:50) and VEGF (1:50) and then washed (3x5 min in TBS). Location of the primary antibodies was achieved by subsequent application for 20 min of the appropriate species-specific biotin-conjugated anti-secondary antibody (a streptavidin-peroxidase (KIT Immunoperoxidase Secondary Detection System, Chemicon, Millipore, Milan ITA). Following washing (3x5 min in TBS), the sections were incubated for 15 min in streptavidin-conjugated HRP. After this incubation, sections were then exposed to 3,3-diaminobenzidine tetrahydrochloride (DAB, Sigma Aldrich, Milan, ITA) for 10 min to produce a brown reaction product. Sections were then counterstained with hematoxylin and mounted in Aquatex (Merck, Milan, ITA). Images were analyzed using Nikon Eclypse T200 at magnification 60x. In all animals, 24 h urine samples were collected at baseline, before the cell implantation, and at day 27, before the sacrifice for the assessment of prostanoid urinary metabolite levels.

*Assessment of urinary levels of PGE-M, PGD-M, PGI-M and TX-M.*

An LC/MS method which allows the simultaneous quantification of tetranor PGE-M and tetranor PGD-M as well as 2,3-dinor-6-keto-PGF1α (PG-IM) and 2,3-dinor-TXB2 (TX-M) was applied. Urine samples (0.2 ml aliquot) were supplemented with tetranor PGEM-d6 (at final concentration of 2 ng/0.2 ml) (Cayman Chemical, Ann Arbor, USA), tetranor PGDM-d6 (2 ng/0.2 ml) (Cayman Chemical, Ann Arbor, USA), 2,3-dinor-6-keto-PGF1α-d9 (sodium salt) (1 ng/0.2 ml) (Cayman Chemical, Ann Arbor, USA), 2,3-dinor-TXB2-d9 (2 ng/0.2 ml) (Cayman Chemical, Ann Arbor, USA) as internal standards, and incubated at room temperature for 15 min. Then, formic acid (5 µl) was added. After 15 minutes of incubation, methoxyamine-HCl (1 g/ml, 0.1 ml) (Sigma-Aldrich, St Louis, USA) was added to generate O-methyl oximes from each ketone group. Following 30 minutes of incubation at room temperature, urine samples were diluted to 1 ml with water, adjusted to pH 3 with HCl, and extracted with Number Strata × 33u polymeric reversed phase (30 mg/ml) (Phenomenex, Torrance, USA) that had been preconditioned with 1 ml acetonitrile and 1 ml water48. The Number Strata × 33u polymeric reversed phase column was washed with 1 ml water (5% acetonitrile) and then eluted with 1 ml of ethyl acetate. The eluate was evaporated and the dried residue was resuspended in 50 µl mobile phase (10% acetonitrile in water), and 40 µl were injected into the LC-MS/MS system and analysed.

### Statistics

Results are given as mean + SEM of at least three independent experiments. Statistical analysis was carried out by Student's paired or unpaired t-test (two-tailed), respectively, for the time course experiments with 2-way ANOVA (Bonferroni post test) using GraphPad Prism 5.0. Differences were considered as significant for p<0.05 (indicated as ^{∗} for p<0.05, ^{∗∗} for p<0.01 and ^{∗∗∗}for p<0.001). Mouse experiments were analyzed by ANOVA analysis and Newman-Keuls multiple comparison tests (§p<0.05, ^{∗}p<0.01, ^{∗∗}p<0.001).

### RNA immunoprecipitation

3×106 A549 cells were seeded in two 10-cm dishes and cultured overnight, before stimulation with 5 ng/ml IL-1β for 24 h. Then, cells were washed with 5 ml ice-cold PBS and harvest was conducted with a cell scraper in 5 ml PBS with protease inhibitor EDTA-free (Roche, Basel, CHE). Cells were pooled, spinned down for 5 min and 400 × g at 4°C and resuspended in 1 ml lysis buffer containing 10 mM Tris-HCl (Carl Roth, Karlsruhe, GER) pH 7.5, 10 mM KCl (Sigma-Aldrich, Darmstadt, GER), 1.5 mM MgCl2, 0.5 mM DTT (Sigma-Aldrich, Darmstadt, GER), 0.9% Nonidet P-40 (Sigma-Aldrich, Darmstadt, GER), 20 µl ribonuclease inhibitor and protease inhibitor EDTA-free (Roche, Basel, CHE). The suspension was incubated 10 min on ice and then sonicated 4 times for 10 sec on 30% amplitude, with 20 sec pause. Afterwards, samples were spinned down with 10,000 × g for 10 min at 4°C to remove cell debris. The supernatant was transferred into a fresh tube and 10% were taken as input sample.

GammaBind Plus Sepharose beads (GE Healthcare, Freiburg, GER) were blocked with blocking buffer containing 0.2 mg/ml BSA in PBS (Sigma-Aldrich, Darmstadt, GER) and 0.1 mg/ml yeast tRNA for 90 min at 4°C. Before usage the beads were washed 3 times with lysis buffer and centrifuged at 300 × g for 5 min. Beads and antibodies were linked by mixing 50 µl bead suspension with 10 µg of CUGBP1 antibody (05-621 clone3B1, Merck, Darmstadt, GER) or normal mouse IgG antibody (12-371, Merck, Darmstadt, GER) followed by incubation for 30-60 min at 4°C. Immunoprecipitation was then conducted by dividing the lysate equally to the CUGBP1- /IgG-bead mixture and incubating for 2 h at 4°C. Afterwards, samples were washed with each wash buffer B1-B3 (composition see below) for 5 min in the cold room, with centrifugation steps of 5 min and 300 × g in between. Wash buffers contained: B1 buffer - 20mM Tris-HCl (Sigma-Aldrich, Darmstadt, GER) pH 7.5, 150 mM NaCl (Sigma-Aldrich, Darmstadt, GER), 2 mM EDTA (Sigma-Aldrich, Darmstadt, GER), 0.1% SDS (Carl Roth, Karlsruhe, GER), 1% Triton (Carl Roth, Karlsruhe, GE) and protease inhibitor EDTA-free (Roche, Basel, CHE). B2 buffer - 20 mM Tris-HCl (Sigma-Aldrich, Darmstadt, GER) pH 7.5, 500 mM NaCl (Sigma-Aldrich, Darmstadt, GER), 2 mM EDTA (Sigma-Aldrich, Darmstadt, GER), 0.1% SDS (Carl Roth, Karlsruhe, GER), 1% Triton (Carl Roth, Karlsruhe, GE) and protease inhibitor EDTA-free (Roche, Basel, CHE). B3 buffer - 10 mM Tris-HCl (Sigma-Aldrich, Darmstadt, GER) pH 7.5, 250 mM LiCl (Sigma-Aldrich, Darmstadt, GER), 1 mM EDTA (Sigma-Aldrich, Darmstadt, GER), 1% sodium deoxycholate (Sigma-Aldrich, Darmstadt, GER), 1% Nonidet P-40 (Sigma-Aldrich, Darmstadt, GER), and protease inhibitor EDTA-free (Roche, Basel, CHE). After the last wash step, 10% of each precipitate was taken for Western blot analysis to validate the immunoprecipitation. The remaining precipitates were resuspended in 500 µl TRIzol reagent (Invitrogen, Karlsruhe, GER) and RNA was isolated as described above. Subsequent qRT-PCR based miR-574-5p or mRNA quantification was performed as previously described. Primers for mPGES-1 CDS (mPGES-1-cds_F: GAAGAAGGCCTTTGCCAAC; mPGES-1-cds_R: CCAGGAAAAGGAAGGGGTAG), COX-2 (COX-2_F: CCGGGTACAATCGCACTTAT; COX-2_R: GGCGCTCAGCCATACAG); miR-574-5p (MS00043617, Qiagen), Hilden, GER or miR-16 (MS00031493, Qiagen, Hilden, GER ) were used.

### Example 12: CUGBP1 binds to the GREs in the mPGES-1 3'UTR and to miR-574-5p

In order to further confirm binding of mPGES1 mRNA as well as miR-574-5p to CUGBP1, RNA immunoprecipitation (RIP) assays with an antibody directed against CUGBP1 were perfomed (Fig. 7 a,b). Thereby, co-precipitated RNAs bound to CUGBP1 can be quantified via qRT-PCR. Validation of the CUGBP1 immunoprecipitation by Western Blot revealed that 16.6% ± 7.7 (SEM) of the total CUGBP1 were recovered in the immune precipitates (Fig. 7c). A representative blot of four independent experiments is shown in Fig. Xc. We found that miR-574-5p as well as mPGES-1 mRNA is highly enriched in the CUGBP1-RIP (Fig. 7a and 7b, respectively). No enrichment was observed for COX-2 mRNA or miR-16 that served as negative controls, respectively. This further confirms the specific interaction of CUGBP1 with miR-574-5p and mPGES-1 mRNA. RNA immunoprecipitation (RIP) assays were performed to confirm the interaction between mPGES-1 3'UTR and miR-574-5p.

### Example 13: mPGES-1 and CUGBP1 expression determines survival rate in NSCLC

We analyzed mPGES-1 and CUGBP1 expression in correlation to the overall survival of lung cancer patients, using the "Kaplan-Meier Plotter" meta-analysis tool (http://kmplot.com/analysis/; version 2015). The overall survival analysis was performed on gene array data from 1926 lung cancer patients. The optimal microarray probe set was selected to represent the best specific probes that recognized mPGES-1 and CUGBP1. The samples were divided into two groups (high and low expression) using the medium gene expression value and were compared using Kaplan-Meier survival plot. The Kaplan-Meier plots show that lung cancer patients with a high expression level of mPGES-1 and a low expression of CUGBP1 are associated with a shorter survival rate (Fig. 8a,b).

### Cited literature

Pai, R., et al. Prostaglandin E2 transactivates EGF receptor: a novel mechanism for promoting colon cancer growth and gastrointestinal hypertrophy. Nat Med 8, 289-293 (2002).
Jakobsson, P.J., Thoren, S., Morgenstern, R. & Samuelsson, B. Identification of human prostaglandin E synthase: a microsomal, glutathione-dependent, inducible enzyme, constituting a potential novel drug target. Proc Natl Acad Sci U S A 96, 7220-7225 (1999).
Patrignani, P. & Patrono, C. Cyclooxygenase inhibitors: From pharmacology to clinical readouts. Biochim Biophys Acta 1851, 422-432 (2015).
Hughes, D., et al. NAD+-dependent 15-hydroxyprostaglandin dehydrogenase regulates levels of bioactive lipids in non-small cell lung cancer. Cancer Prev Res (Phila) 1, 241-249 (2008).
Leclerc, P., et al. Characterization of a new mPGES-1 inhibitor in rat models of inflammation. Prostaglandins Other Lipid Mediat 102-103, 1-12 (2013).
Li, Q., et al. MicroRNA-574-5p was pivotal for TLR9 signaling enhanced tumor progression via down-regulating checkpoint suppressor 1 in human lung cancer. PLoS One 7, e48278 (2012).
Zhou, R., et al. MicroRNA-574-5p promotes metastasis of non-small cell lung cancer by targeting PTPRU. Sci Rep 6, 35714 (2016).

## Claims

1. A method for identifying a subject suffering from a prostaglandin E-dependent tumor as being susceptible to a therapy with an inhibitor of prostaglandin E formation comprising the steps of:
a) determining in a test sample of the subject the amount of microRNA-574-5p;
b) comparing the determined amount to a reference; and
c) identifying a subject suffering from a prostaglandin E-dependent tumor as being susceptible to a therapy with an inhibitor of prostaglandin E formation based on said comparison.

2. The method of claim 1, wherein said reference is derived from a subject or a group of subjects suffering from a prostaglandin E-dependent tumor known to be susceptible to a therapy with an inhibitor of prostaglandin E formation.

3. The method of claim 2, wherein an essentially identical or increased amount of microRNA-574-5p in the test sample is indicative for a subject being susceptible to a therapy with an inhibitor of prostaglandin E formation and wherein a decreased amount of microRNA-574-5p in the test sample is indicative for a subject being not susceptible to a therapy with an inhibitor of prostaglandin E formation.

4. The method of claim 1, wherein said reference is derived from (i) a non-tumor tissue sample or from (ii) a subject or a group of subjects suffering from a prostaglandin E-dependent tumor known not to be susceptible to a therapy with an inhibitor of prostaglandin E formation or (iii) a healthy subject or group of healthy subjects.

5. The method of claim 4, wherein an essentially identical or decreased amount of microRNA-574-5p in the test sample is indicative for a subject being not susceptible to a therapy with an inhibitor of prostaglandin E formation and wherein an increased amount of microRNA-574-5p in the test sample is indicative for a subject being susceptible to a therapy with an inhibitor of prostaglandin E formation.

6. The method of any one of claims 1 to 5, wherein said sample is a tissue sample or a body fluid sample.

7. The method of claim 6, wherein said body fluid sample is selected from the group consisting of: whole blood, serum, plasma, urine and lymphe.

8. The method of any one of claims 1 to 7, wherein said tumor is selected from the group consisting of: lung cancer, colon carcinoma, breast cancer, prostate cancer, head and neck cancers, papillary thyroid carcinoma, low and high risk gliomas, neuroblastoma, medulloblastoma, cervix carcinoma, brain tumors and stomach cancer.

9. The method of any one of claims 1 to 8, wherein said subject is a mammal and, preferably, a human.

10. The method of any one of claims 1 to 9, wherein said inhibitor of prostaglandin E formation is a non-steroidal anti-inflammatory drug (NSAID) and/or an inhibitor of microsomal prostaglandin E-synthase-1 (mPGES1 inhibitor).

11. The method claim 10, wherein said NSAID is selected from the group consisting of: selective COX-2 inhibitors, preferably, parecoxib, etoricoxib, or celecoxib, COX1-selective inhibitors, preferably, low dose Aspirin, and non-selective COX1/2 inhibitors, preferably, naproxen, ibuprofen or diclofenac.

12. The method of claim 10, wherein said mPGES1 inhibitor is selected from the group consisting of: MK-886 derivatives, phenanthrene or benzo imidazoles, biarylimidazoles, pirinixic acid derivatives, 2-mercaptohexanoic acid, licofelone derivatives, benzo(g)indol-3-carboxylate, oxicams, trisubstituted ureas, and carbazole benzamides.

13. Use of microRNA-574-5p or a detection agent that specifically binds thereto in a sample of a subject suffering from a prostaglandin E-dependent tumor for identifying the subject as being susceptible to a therapy with an inhibitor of prostaglandin E formation.

14. A device adapted for carrying out the method of any one of claims 1 to 12 comprising
a) an analyzing unit comprising a detection agent which specifically binds to microRNA-574-5p adapted for determining the amount of said microRNA-574-5p in a sample of a subject suffering from a prostaglandin E-dependent tumor; and
b) an evaluation unit for comparing the determined amount with a reference whereby the subject can be identified as being susceptible to a therapy with an inhibitor of prostaglandin E formation, said unit comprising a database with references as defined in claim 2 or 4 and a computer-implemented algorithm for carrying out a comparison.

15. An inhibitor of prostaglandin E formation for use in treating a prostaglandin E-dependent tumor in a subject, wherein said subject exhibits increased amounts of miRNA-574-5p (i) in the tumor tissue compared to non-tumor tissue or (ii) in a body fluid of the subject compared to a reference from a healthy subject or a group of healthy subjects or a subject known to not suffer from a prostaglandin E-dependent tumor or a group of such subjects, preferably, wherein said subject was identified as being susceptible to a therapy with an inhibitor of prostaglandin E formation by the method according to any one of claims 1 to 12.

## Patentansprüche

1. Verfahren zum Erkennen, dass ein an einem prostaglandin-E-abhängigen Tumor leidendes Individuum einer Therapie mit einem Inhibitor der Prostaglandin-E-Bildung zugänglich ist, umfassend die Schritte:
a) Bestimmen der Menge an microRNA-574-5p in einer Testprobe vom Individuum;
b) Vergleichen der bestimmten Menge mit einer Referenz; und
c) Erkennen anhand des Vergleichs, dass ein an einem prostaglandin-E-abhängigen Tumor leidendes Individuum einer Therapie mit einem Inhibitor der Prostaglandin-E-Bildung zugänglich ist.

2. Verfahren nach Anspruch 1, wobei die Referenz von einem Individuum oder einer Gruppe von Individuen stammt, das/die an einem prostaglandin-E-abhängigen Tumor leidet/leiden, der bekanntermaßen einer Therapie mit einem Inhibitor der Prostaglandin-E-Bildung zugänglich ist.

3. Verfahren nach Anspruch 2, wobei eine im Wesentlichen identische oder erhöhte Menge an microRNA-574-5p in der Testprobe anzeigt, dass das Individuum einer Therapie mit einem Inhibitor der Prostaglandin-E-Bildung zugänglich ist, und wobei eine verminderte Menge an microRNA-574-5p in der Testprobe anzeigt, dass das Individuum einer Therapie mit einem Inhibitor der Prostaglandin-E-Bildung nicht zugänglich ist.

4. Verfahren nach Anspruch 1, wobei die Referenz von (i) einer Nichttumor-Gewebeprobe oder von (ii) einem Individuum oder einer Gruppe von Individuen, das/die an einem prostaglandin-E-abhängigen Tumor leidet/leiden, der bekanntermaßen einer Therapie mit einem Inhibitor der Prostaglandin-E-Bildung nicht zugänglich ist, oder (iii) einem gesunden Individuum oder einer Gruppe von gesunden Individuen stammt.

5. Verfahren nach Anspruch 4, wobei eine im Wesentlichen identische oder verminderte Menge an microRNA-574-5p in der Testprobe anzeigt, dass das Individuum einer Therapie mit einem Inhibitor der Prostaglandin-E-Bildung nicht zugänglich ist, und wobei eine erhöhte Menge an microRNA-574-5p in der Testprobe anzeigt, dass das Individuum einer Therapie mit einem Inhibitor der Prostaglandin-E-Bildung zugänglich ist.

6. Verfahren nach einem der Ansprüche 1 bis 5, wobei es sich bei der Probe um eine Gewebeprobe oder eine Körperflüssigkeitsprobe handelt.

7. Verfahren nach Anspruch 6, wobei die Körperflüssigkeitsprobe ausgewählt ist aus der Gruppe bestehend aus: Vollblut, Serum, Plasma, Urin und Lymphe.

8. Verfahren nach einem der Ansprüche 1 bis 7, wobei der Tumor ausgewählt ist aus der Gruppe bestehend aus: Lungenkrebs, Kolonkarzinom, Brustkrebs, Prostatakrebs, Kopf-Hals-Krebs, papillärem Schilddrüsenkarzinom, Gliomen mit geringem und hohem Risiko, Neuroblastom, Medulloblastom, Zervixkarzinom, Hirntumoren und Magenkrebs.

9. Verfahren nach einem der Ansprüche 1 bis 8, wobei es sich bei dem Individuum um einen Säuger und bevorzugt einen Menschen handelt.

10. Verfahren nach einem der Ansprüche 1 bis 9, wobeies sich bei dem Inhibitor der Prostaglandin-E-Bildung um einen nichtsteroidalen entzündungshemmenden Arzneistoff (Non-steroidal Anti-inflammatory Drug, NSAID) und/oder einen Inhibitor von mikrosomaler Prostaglandin-E-Synthase-1 (mPGES1-Inhibitor) handelt.

11. Verfahren nach Anspruch 10, wobei der NSAID ausgewählt ist aus der Gruppe bestehend aus: selektiven COX-2-Inhibitoren, bevorzugt Parecoxib, Etoricoxib oder Celecoxib, COX1-selektiven Inhibitoren, bevorzugt Aspirin in geringer Dosis, und nicht selektiven COX1/2-Inhibitoren, bevorzugt Naproxen, Ibuprofen oder Diclofenac.

12. Verfahren nach Anspruch 10, wobei der mPGES1-Inhibitor ausgewählt ist aus der Gruppe bestehend aus: MK-886-Derivaten, Phenanthren oder Benzimidazolen, Biarylimidazolen, Pirinixinsäurederivaten, 2-Mercaptohexansäure, Licofelonderivaten, Benzo(g)-indol-3-carboxylat, Oxicamen, trisubstituierten Harnstoffen und Carbazolbenzamiden.

13. Verwendung von microRNA-574-5p oder einem Nachweismittel, das daran spezifisch bindet, in einer Probe von einem an einem prostaglandin-E-abhängigen Tumor leidenden Individuum zum Erkennen, dass das Individuum einer Therapie mit einem Inhibitor der Prostaglandin-E-Bildung zugänglich ist.

14. Vorrichtung, eingerichtet zur Durchführung des Verfahrens nach einem der Ansprüche 1 bis 12, umfassend
a) eine Analysiereinheit, die ein Nachweismittel umfasst, das an microRNA-574-5p spezifisch bindet, eingerichtet zur Bestimmung der Menge der microRNA-574-5p in einer Probe von einem an einem prostaglandin-E-abhängigen Tumor leidenden Individuum; und
b) eine Auswertungseinheit zum Vergleichen der bestimmten Menge mit einer Referenz, wodurch sich erkennen lässt, dass das Individuum einer Therapie mit einem Inhibitor der Prostaglandin-E-Bildung zugänglich ist, wobei die Einheit eine Datenbank mit Referenzen gemäß Anspruch 2 oder 4 und einen computerimplementierten Algorithmus zur Durchführung eines Vergleichs umfasst.

15. Inhibitor der Prostaglandin-E-Bildung zur Verwendung beim Behandeln eines prostaglandin-E-abhängigen Tumors bei einem Individuum, wobei das Individuum erhöhte Mengen an miRNA-574-5p (i) im Tumorgewebe verglichen mit Nichttumorgewebe oder (ii) in einer Körperflüssigkeit des Individuums verglichen mit einer Referenz aus einem gesunden Individuum oder einer Gruppe gesunder Individuen oder einem Individuum, das bekanntermaßen nicht an einem prostaglandin-E-abhängigen Tumor leidet, oder einer Gruppe solcher Individuen, vorzugsweise, wobei mit dem Verfahren gemäß einem der Ansprüche 1 bis 12 erkannt wurde, dass das Individuum einer Therapie mit einem Inhibitor der Prostaglandin-E-Bildung zugänglich ist.

## Revendications

1. Procédé pour l'identification d'un sujet souffrant d'une tumeur prostaglandine E-dépendante comme étant susceptible à une thérapie avec un inhibiteur de formation de prostaglandine E comprenant les étapes de :
a) détermination dans un échantillon de test du sujet de la quantité de microARN-574-5p ;
b) comparaison de la quantité déterminée à une référence ; et
c) identification d'un sujet souffrant d'une tumeur prostaglandine E-dépendante comme étant susceptible à une thérapie avec un inhibiteur de formation de prostaglandine E sur la base de ladite comparaison.

2. Procédé selon la revendication 1, ladite référence étant issue d'un sujet ou d'un groupe de sujets souffrant d'une tumeur prostaglandine E-dépendante connu pour être susceptible à une thérapie avec un inhibiteur de formation de prostaglandine E.

3. Procédé selon la revendication 2, une quantité essentiellement identique ou augmentée de microARN-574-5p dans l'échantillon de test indiquant qu'un sujet est susceptible à une thérapie avec un inhibiteur de formation de prostaglandine E et une quantité diminuée de microARN-574-5p dans l'échantillon de test indiquant qu'un sujet n'est pas susceptible à une thérapie avec un inhibiteur de formation de prostaglandine E.

4. Procédé selon la revendication 1, ladite référence étant issue (i) d'un échantillon de tissu non tumoral ou (ii) d'un sujet ou d'un groupe de sujets souffrants d'une tumeur prostaglandine E-dépendante connu pour ne pas être susceptible à une thérapie avec un inhibiteur de formation de prostaglandine E ou (iii) d'un sujet sain ou d'un groupe de sujets sains.

5. Procédé selon la revendication 4, une quantité essentiellement identique ou diminuée de microARN-574-5p dans l'échantillon de test indiquant qu'un sujet n'est pas susceptible à une thérapie avec un inhibiteur de formation de prostaglandine E et une quantité augmentée de microARN-574-5p dans l'échantillon de test indiquant qu'un sujet est susceptible à une thérapie avec un inhibiteur de formation de prostaglandine E.

6. Procédé selon l'une quelconque des revendications 1 à 5, ledit échantillon étant un échantillon de tissu ou un échantillon de fluide corporel.

7. Procédé selon la revendication 6, ledit échantillon de fluide corporel étant choisi dans le groupe constitué par : le sang entier, le sérum, le plasma, l'urine et la lymphe.

8. Procédé selon l'une quelconque des revendications 1 à 7, ladite tumeur étant choisie dans le groupe constitué par : un cancer du poumon, un carcinome du côlon, un cancer du sein, un cancer de la prostate, des cancers de la tête et du cou, un carcinome de la thyroïde papillaire, des gliomes à risque faible et à risque élevé, un neuroblastome, un médulloblastome, un carcinome du col de l'utérus, des tumeurs au cerveau et un cancer de l'estomac.

9. Procédé selon l'une quelconque des revendications 1 à 8, ledit sujet étant un mammifère et, préférablement, un être humain.

10. Procédé selon l'une quelconque des revendications 1 à 9, ledit inhibiteur de formation de prostaglandine E étant un médicament anti-inflammatoire non stéroïdien (AINS) et/ou un inhibiteur de prostaglandine E-synthase-1 microsomale (inhibiteur de mPGES1).

11. Procédé selon la revendication 10, ledit NAIS étant choisi dans le groupe constitué par : des inhibiteurs sélectifs de COX-2, préférablement, le parécoxib, l'étoricoxib ou le célécoxib, des inhibiteurs sélectifs de COX-1, préférablement l'aspirine à faible dose, et des inhibiteurs non sélectifs de COX1/2, préférablement, le naproxène, l'ibuprofène ou le diclofénac.

12. Procédé selon la revendication 10, ledit inhibiteur de mPGES1 étant choisi dans le groupe constitué par : des dérivés de MK-886, le phénanthrène ou des benzoimidazoles, des biarylimidazoles, des dérivés d'acide pirinixique, l'acide 2-mercaptohexanoïque, des dérivés de licofélone, un benzo(g)indol-3-carboxylate, des oxicams, des urées trisubstituées et des benzamides de carbazole.

13. Utilisation de microARN-574-5p ou d'un agent de détection qui se lie spécifiquement à celui-ci dans un échantillon d'un sujet souffrant d'une tumeur prostaglandine E-dépendante pour l'identification du sujet comme étant susceptible à une thérapie avec un inhibiteur de formation de prostaglandine E.

14. Dispositif conçu pour mettre en œuvre le procédé selon l'une quelconque des revendications 1 à 12 comprenant
a) une unité d'analyse comprenant un agent de détection qui se lie spécifiquement à microARN-574-5p conçue pour déterminer la quantité dudit microARN-574-5p dans un échantillon d'un sujet souffrant d'une tumeur prostaglandine E-dépendante ; et
b) une unité d'évaluation pour la comparaison de la quantité déterminée avec une référence, moyennant quoi le sujet peut être identifié comme étant susceptible à une thérapie avec un inhibiteur de formation de prostaglandine E, ladite unité comprenant une base de données comportant des références telles que définies dans la revendication 2 ou 4 et un algorithme informatisé pour réaliser une comparaison.

15. Inhibiteur de formation de prostaglandine E pour une utilisation dans le traitement d'une tumeur prostaglandine E-dépendante chez un sujet, ledit sujet présentant des quantités augmentées de miARN-574-5p (i) dans le tissu tumoral par comparaison avec un tissu non tumoral ou (ii) dans un fluide corporel du sujet par comparaison avec une référence provenant d'un sujet sain ou d'un groupe de sujets sains ou d'un sujet dont on sait qu'il ne souffre pas d'une tumeur prostaglandine E-dépendante ou d'un groupe de tels sujets, préférablement, ledit sujet ayant été identifié comme étant susceptible à une thérapie avec un inhibiteur de formation de prostaglandine E par le procédé selon l'une quelconque des revendications 1 à 12.


### SEQUENCE LISTING

<110> Johann Wolfgang Goethe-Universität Frankfurt Fraunhofer-Gesellschaft zur Förderung der angewandten Forschung e.V. Technische Universität Darmstadt
<120> MiRNA-574-5p as a biomarker for stratification of prostaglandin
   E-dependent tumors
<130> FH59330PC
<150> EP17175535.8
   <151> 2017-06-12
<160> 32
<170> BiSSAP 1.3.6
<210> 1
   <211> 39
   <212> RNA
   <213> Homo sapiens
<400> 1
   ugugugugug cccgugugug uguaugugug uguguaugu 39
<210> 2
   <211> 46
   <212> RNA
   <213> Homo sapiens
<400> 2
   gugugugugu gugugugugu gugugugugu gugugugugu gugugu 46
<210> 3
   <211> 23
   <212> RNA
   <213> Homo sapiens
<400> 3
   ugagugugug ugugugagug ugu 23
<210> 4
   <211> 39
   <212> RNA
   <213> Artificial Sequence
<220>
   <223> olignonculeotide
<400> 4
   ggucacgguc acggucacgg ucacggucac ggucacggu 39
<210> 5
   <211> 19
   <212> RNA
   <213> Artificial Sequence
<220>
   <223> oligonucleotide
<400> 5
   gcuguuuauu gguaugauu 19
<210> 6
   <211> 19
   <212> RNA
   <213> Artificial Sequence
<220>
   <223> oligonucleotide
<400> 6
   ucucucacaa cgggcauuu 19
<210> 7
   <211> 21
   <212> DNA
   <213> Artificial Sequence
<220>
   <223> primer
<400> 7
   ccagcagctg atgcctcctt g 21
<210> 8
   <211> 21
   <212> DNA
   <213> Artificial Sequence
<220>
   <223> primer
<400> 8
   gcccagctgg cagacacttc c 21
<210> 9
   <211> 29
   <212> DNA
   <213> Artificial Sequence
<220>
   <223> primer
<400> 9
   aagcggccgc ccagcagctg atgcctcct 29
<210> 10
   <211> 24
   <212> DNA
   <213> Artificial Sequence
<220>
   <223> primer
<400> 10
   aagctttgct gggcccagct ggca 24
<210> 11
   <211> 39
   <212> DNA
   <213> Artificial Sequence
<220>
   <223> primer
<400> 11
   tccccttgat ggggaatcct ttctcctaga cccgtgacc 39
<210> 12
   <211> 34
   <212> DNA
   <213> Artificial Sequence
<220>
   <223> primer
<400> 12
   cgggtctagg agaaaggatt ccccatcaag ggga 34
<210> 13
   <211> 55
   <212> DNA
   <213> Artificial Sequence
<220>
   <223> primer
<400> 13
   gcgcgtgtgg gtctctgggc acagtgggcc ttttcttagc cccttggatt cctgc 55
<210> 14
   <211> 45
   <212> DNA
   <213> Artificial Sequence
<220>
   <223> primer
<400> 14
   gcaggaatcc aaggggctaa gaaaaggccc actgtgccca gagac 45
<210> 15
   <211> 20
   <212> DNA
   <213> Artificial Sequence
<220>
   <223> primer
<400> 15
   aatctggtgc agcaaactcc 20
<210> 16
   <211> 20
   <212> DNA
   <213> Artificial Sequence
<220>
   <223> primer
<400> 16
   agtgttttgt gcaggggttc 20
<210> 17
   <211> 20
   <212> DNA
   <213> Artificial Sequence
<220>
   <223> primer
<400> 17
   aaagtcctcc cagggatgca 20
<210> 18
   <211> 22
   <212> DNA
   <213> Artificial Sequence
<220>
   <223> primer
<400> 18
   agcttcctgt cttccactgc at 22
<210> 19
   <211> 29
   <212> DNA
   <213> Artificial Sequence
<220>
   <223> primer
<400> 19
   tgcccgtgtg tgtgtatgtg tgtgtgtgt 29
<210> 20
   <211> 31
   <212> DNA
   <213> Artificial Sequence
<220>
   <223> primer
<400> 20
   cccagctggc agacacttcc atttaatgac t 31
<210> 21
   <211> 20
   <212> DNA
   <213> Artificial Sequence
<220>
   <223> primer
<400> 21
   ccgggtacaa tcgcacttat 20
<210> 22
   <211> 17
   <212> DNA
   <213> Artificial Sequence
<220>
   <223> primer
<400> 22
   ggcgctcagc catacag 17
<210> 23
   <211> 18
   <212> DNA
   <213> Artificial Sequence
<220>
   <223> primer
<400> 23
   tcccgggcta agaatgca 18
<210> 24
   <211> 20
   <212> DNA
   <213> Artificial Sequence
<220>
   <223> primer
<400> 24
   attggctggg ccagaatttc 20
<210> 25
   <211> 18
   <212> DNA
   <213> Artificial Sequence
<220>
   <223> primer
<400> 25
   cgggacctga ctgactac 18
<210> 26
   <211> 22
   <212> DNA
   <213> Artificial Sequence
<220>
   <223> primer
<400> 26
   cttctcctta atgtcacgca cg 22
<210> 27
   <211> 49
   <212> DNA
   <213> Artificial Sequence
<220>
   <223> primer
<400> 27
   aaaaaagaat tctaatacga ctcactatag gtgtgtgtgt gcccgtgtg 49
<210> 28
   <211> 64
   <212> DNA
   <213> Artificial Sequence
<220>
   <223> primer
<400> 28
   ttttttccat ggccggcaca tacacacaca catacacaca cacgggcaca cacacaccta 60
tagt 64
<210> 29
   <211> 46
   <212> DNA
   <213> Artificial Sequence
<220>
   <223> primer
<400> 29
   aaaaaagaat tctaatacga ctcactatag ggtgtgtgtg tgtgtg 46
<210> 30
   <211> 72
   <212> DNA
   <213> Artificial Sequence
<220>
   <223> primer
<400> 30
   ttttaccatg gccggccaca cacacacaca cacacacaca cacacacaca cacacacaca 60
caccctatag tg 72
<210> 31
   <211> 19
   <212> DNA
   <213> Artificial Sequence
<220>
   <223> mPGES-1-cds_F
<400> 31
   gaagaaggcc tttgccaac 19
<210> 32
   <211> 20
   <212> DNA
   <213> Artificial Sequence
<220>
   <223> mPGES-1-cds_R
<400> 32
   ccaggaaaag gaaggggtag 20